# EUROPÄISCHE PATENTSCHRIFT

(11) **EP 0 497 176 B1**
(45) Veröffentlichungstag und Bekanntmachung des Hinweises auf die Patenterteilung: **05.04.2000**
(21) Anmeldenummer: 92100837.1
(22) Anmeldetag: 20.01.1992
(51) Int. Cl.: C07D 319/06, C09K 19/34, C09K 19/30, C07D 239/26, C07D 213/24, C07C 25/18, C07C 43/12, G02F 1/1337

(54) **Vierringverbindungen und sie enthaltende flüssigkristalline Gemische**
Four rings compounds and liquid crystal mixtures containing them
Composés à quatre cycles et mélanges de cristaux liquides les contenant

(30) Priorität: 01.02.1991 CH 30591
(43) Veröffentlichungstag der Anmeldung: 05.08.1992
(73) Patentinhaber: MERCK PATENT GmbH, 64293 Darmstadt (DE)
(72) Erfinder: Buchecker, Richard, CH-8008 Zürich (CH); Schadt, Martin, CH-4411 Seltisberg (CH); Villiger, Alois, CH-4055 Basel (CH)

(56) Entgegenhaltungen:
- EP-A- 0 291 949
- EP-A- 0 315 014
- EP-A- 0 391 591
- WO-A-91/03450
- WO-A-91/11497
- WO-A-91/16394
- WO-A-91/17134
- CHEMICAL ABSTRACTS, vol. 101, no. 26, 24. Dezember 1984, Columbus, Ohio, US; abstract no. 238500, Seite 505 ;
- CHEMICAL ABSTRACTS, vol. 101, no. 23, 3. Dezember 1984, Columbus, Ohio, US; abstract no. 210757, Seite 615 ;

## Beschreibung

Die vorliegende Erfindung betrifft neue Gemische, enthaltend Vierringverbindungen sowie die Verwendung dieser Gemische für elektrooptische Zwecke.

Flüssige Kristalle werden vor allem als Dielektrika in Anzeigevorrichtungen eingesetzt, da die optischen Eigenschaften solcher Substanzen durch eine angelegte Spannung beeinflusst werden können. Elektro-optische Vorrichtungen auf der Basis von Flüssigkristallen sind dem Fachmann bestens bekannt und können auf verschiedenen Effekten beruhen. Beispiele solcher Vorrichtungen sind Zellen mit dynamischer Streuung, DAP-Zellen (Deformation aufgerichteter Phasen), Gast/Wirt-Zellen, TN-Zellen mit verdrillt nematischer ("twisted nematic") Struktur, STN-Zellen ("super-twisted nematic"), SBE-Zellen ("super-birefringence effect") und OMI-Zellen ("optical mode interference"). Die gebräuchlichsten Anzeigevorrichtungen beruhen auf dem Schadt-Helfrich-Effekt und besitzen eine verdrillt nematische Struktur.

Die Flüssigkristallmaterialien müssen eine gute chemische und thermische Stabilität und eine gute Stabilität gegenüber elektrischen Feldern und elektromagnetischer Strahlung besitzen. Ferner sollten die Flüssigkristallmaterialien niedere Viskosität aufweisen und in den Zellen kurze Ansprechzeiten, tiefe Schwellenspannung und einen hohen Kontrast ergeben. Weiterhin sollten sie bei üblichen Betriebstemperaturen von etwa -30°C bis etwa +80°C, insbesondere von etwa -20°C bis etwa +60°C, eine geeignete Mesophase besitzen, beispielsweise für die oben genannten Zellen eine nematische oder cholesterische Mesophase. Weitere Eigenschaften, wie die elektrische Leitfähigkeit, die dielektrische Anisotropie und die optische Anisotropie, müssen je nach Zellentyp und Anwendungsbereich unterschiedlichen Anforderungen genügen. Beispielsweise sollten Materialien für Zellen mit verdrillt nematischer Struktur eine positive dielektrische Anisotropie und eine möglichst geringe elektrische Leitfähigkeit aufweisen. Seit einigen jahren besteht ein besonderes Interesse an aktiv adressierten Flüssigkristallanzeigen, z.B. TFT-Anwendungen ("thin film transistor" = Dünnfilmtransistor) in Fernsehgeräten. Die Verwendung von Cyano-Verbindungen mit positiver dielektrischer Anisotropie führt jedoch in solchen Anzeigen meist zu einem unerwünscht hohen Stromanstieg.

Da Flüssigkristalle in der Regel als Mischungen mehrerer Komponenten zur Anwendung gelangen, ist es wichtig, dass die Komponenten untereinander gut mischbar sind.

Gegenstand der Erfindung sind flüssigkristalline Gemische mit mindestens 2 Komponenten, dadurch gekennzeichnet, daß mindestens eine Komponente eine Verbindung der allgemeinen Formel I ist 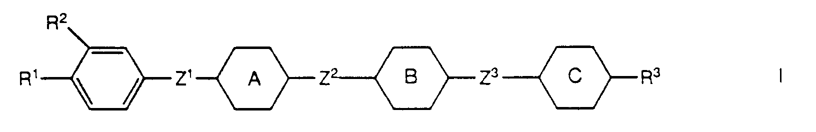 worin
die Ringe A und B unabhängig voneinander trans-1,4-Cyclohexylen, unsubstituiertes oder mit Halogen und/oder Cyano und/oder Methyl mono- oder disubstituiertes 1,4-Phenylen, Pyridin-2,5-diyl oder Pyrimidin-2,5-diyl,
der Ring C trans-1,4-Cyclohexylen oder trans-1,3-Dioxan-2,5-diyl, Z¹ und Z² unabhängig voneinander eine einfache Bindung oder, falls mit mindestens einem gesättigten Ring verknüpft, auch Aethylen,
Z³ eine einfache Bindung oder Aethylen,
R¹ Fluor oder Chlor,
R² Wasserstoff oder Fluor
   und
R³ C₂₋₁₂-1E-Alkenyl, C₄₋₁₂-3E-Alkenyl, C₅₋₁₂-4-Alkenyl oder C₂₋₁₂-Alkoxyalkyl bedeuten, wobei im Falle von Alkoxyalkyl sich nicht mehr als 5 Methylengruppen zwischen dem Sauerstoffatom und dem Ring C befinden,
   mit den Massgaben, dass
   (a) falls der Ring B (hetero)aromatisch ist, der Ring A nicht gleichzeitig trans-1,4-Cyclohexylen ist,
   (b) höchstens zwei mit je einer einfachen Bindung verknüpfte trans-1,4-Cyclohexylengruppen vorhanden sind,
   (c) höchstens einer der Ringe A und B Pyridin-2,5-diyl oder Pyrimidin-2,5-diyl ist, und
   (d) falls gleichzeitig R¹ Fluor und R³ C₂₋₁₂-1E-Alkenyl oder C₄₋₁₂-3E-Alkenyl bedeuten, R² ausschliesslich für Fluor steht,
wobei der Anteil an Verbindungen der Formel I 2-30 Gew.-% beträgt,
und, daß sie eine oder mehrere Verbindungen aus der Gruppe der Verbindungen der allgemeinen Formeln 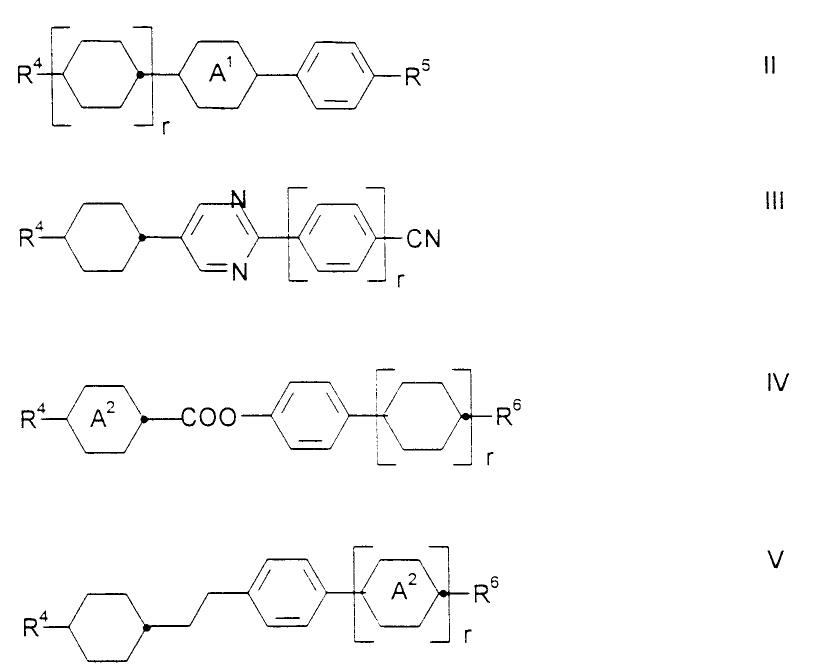 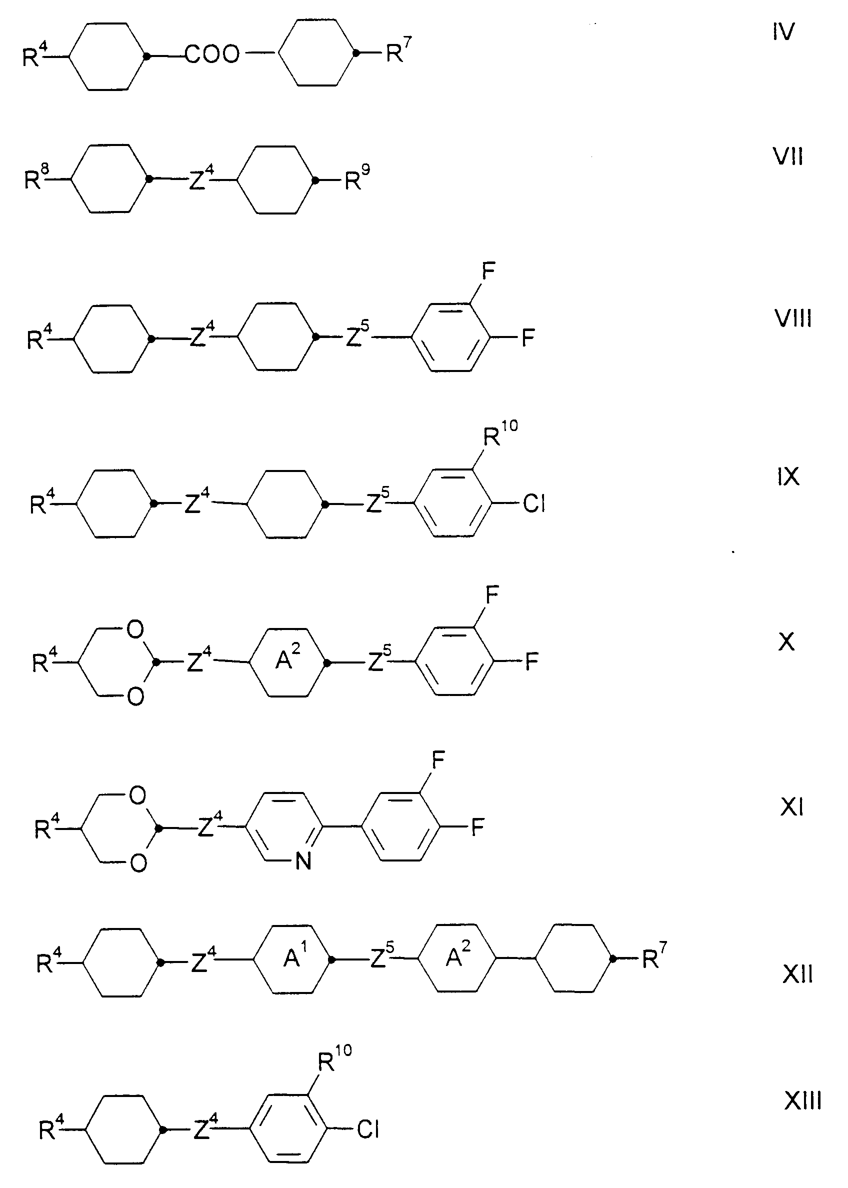 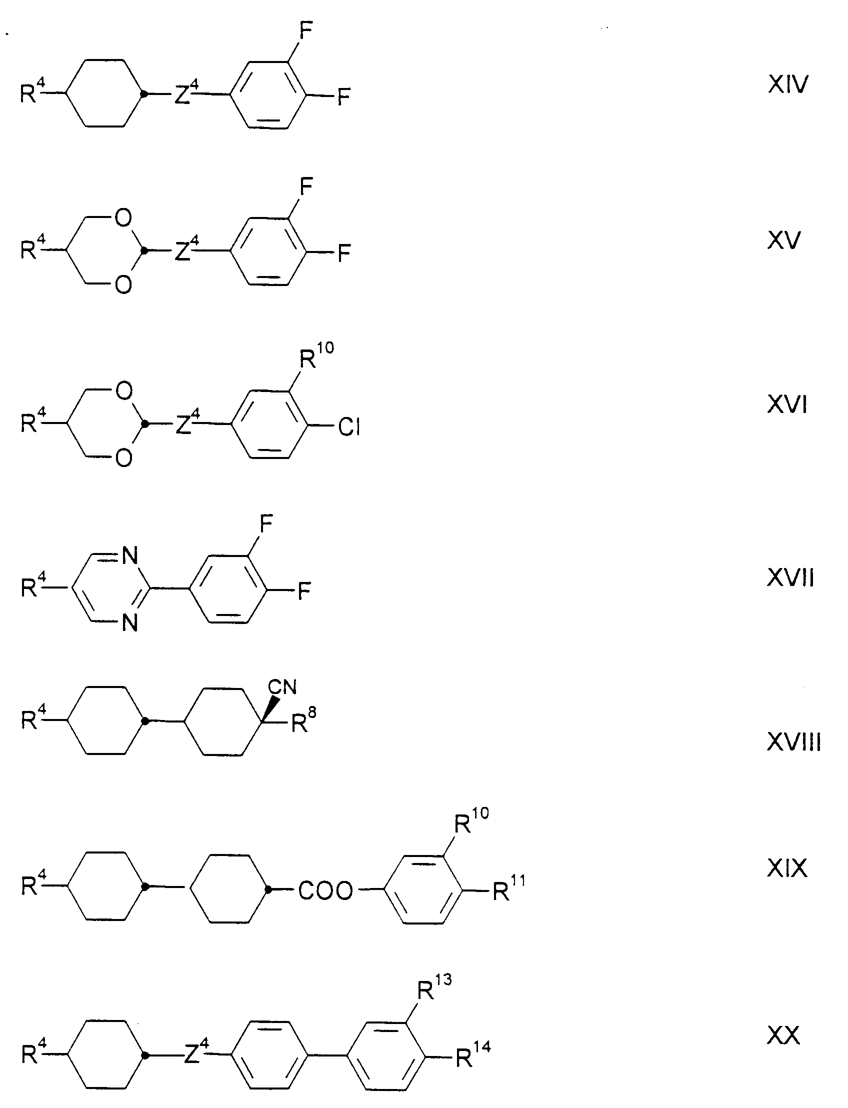 worin
- r: für die Zahl 0 oder 1 steht;
- R⁴ und R⁷: unabhängig voneinander Alkyl, Alkoxylalkyl, 3E-Alkenyl, 4-Alkenyl oder an einem gesättigten Ring auch 1E-Alkenyl bedeutet;
- Ring A¹: 1,4-Phenylen, trans-1,4-Cyclohexylen, Pyridin-2,5-diyl, Pyrimidin-2,5-diyl oder trans-1,3-Dioxan-2,5-diyl darstellt;
- R⁵: Cyano, Isothiocyanato, Fluor, Alkyl, 3E-Alkenyl, 4-Alkenyl, Alkoxy, 2E-Alkenyloxy, 3-Alkenyloxy oder 1-Alkinyl bezeichnet;
- Ring A²: 1,4-phenylen oder trans-1,4-Cyclohexylen darstellt;
- R⁶: Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet;
- R⁸: Alkyl, 1E-Alkenyl, 3E-Alkenyl oder 4-Alkenyl bezeichnet;
- Z⁴ und Z⁵: jeweils eine einfache Bindung oder Aethylen darstellt, wobei zwei aromatische Ringe nur durch eine einfache Bindung gebunden sind;
- R⁹: Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet;
- R¹⁰: Wasserstoff, Fluor oder Chlor bezeichnet;
- R¹¹: Fluor, Chlor oder Cyano bedeutet;
- R¹²: Alkyl, 1E-Alkenyl, 3E-Alkenyl, 3E-Alkenyl oder 4-Alkenyl darstellt;
- R¹³: Wasserstoff oder Fluor bedeutet;
und
- R¹⁴: Fluor oder Chlor bezeichnet,
enthalten.

Die Verbindungen der Formel I sind Flüssigkristalle mit breiten nematischen Phasen und sehr hohen Klärpunkten. Zugleich besitzen sie überraschend kurze Schaltzeiten, insbesondere in Anzeigevorrichtungen mit verdrillt nematischer Struktur. Sie sind niederviskos und gut löslich in üblichen Flüssigkristallmaterialien. Insbesondere erhöhen sie stark den Klärpunkt von Flüssigkristallmischungen und verbessern somit die nematische Mesophase, ohne gleichzeitig die Schwellenspannung heraufzusetzen und die Schaltzeiten unzulässig zu verlängern.

Die Verbindungen der Formel I eignen sich insbesondere als Komponenten nematischer und cholesterischer Gemische und können dank ihrer guten Mischbarkeit untereinander und mit bekannten Flüssigkristallmaterialien in vergleichsweise hohen Konzentrationen verwendet werden. Es hat sich herausgestellt, dass die Verbindungen sehr vorteilhaft für TFT-Anwendungen geeignet sind.

Flüssigkristalline Medien, die der Formel I ähnliche Verbindungen enthalten, sind der Literatur entnehmbar.

EP 0 315 014 A2 offenbart u. a. flüssigkristalline Medien, die Zwei- und Dreiringverbindungen mit Alkenyl- oder Alkenyloxyresten enthalten. Die erfindungsgemäßen Vierringverbindungen enthaltenden Mischungen werden darin jedoch nicht erwähnt.

In EP 0 291 949 A2 und EP 0 391 591 A2 werden Vierringverbindungen beschrieben, die als endständigen Rest eine geradkettige Alkylgruppe aufweisen und als Komponenten flüssigkristalliner Medien dienen können. Ein Hinweis auf Mischungen, die Vierringverbindungen mit Alkenyl- oder Alkoxyalkyl-Gruppen enthalten, wird dem Leser jedoch vorenthalten.

Die Druckschriften JP 59,128,354 und JP 95,108,743 offenbaren Vierkernester, die eine Alkenyloxygruppe aufweisen. Die dort beschriebenen Verbindungen weisen jedoch unerwünschte smektische Phasen auf, besitzen ungünstige Werte für die elastischen Konstanten und verschlechtern die Werte für die Holding Ratio einer LC-Mischung drastisch.

In den Dokumenten WO 91/16394 und WO 91/11497 wird zwar die Verwendung von Alkenylverbindungen in flüssigkristallinen Medien genannt, jedoch wird weder ein Mischungskonzept vorgestellt noch eine Aussage darüber gemacht, welche Alkenylverbindungen (Z- oder E-Alkenyle) die flüssigkristallinen Mischungen positiv beeinflussen. Die in den angegebenen Mischungsbeispielen verwendeten Vierkernverbindungen weisen ausschließlich Alkylreste auf.

In der obigen Definition der Verbindungen I umfassen die Ausdrücke "1E-Alkenyl", "3E-Alkenyl" und "4-Alkenyl" für R³ ausschliesslich Alkenylgruppen, in denen jeweils eine einzige Doppelbindung vorhanden ist, wobei sich diese in der 1,2-, 3,4- bzw. 4,5-Stellung befindet. Darüber hinaus ist der Teil der 1E-, 3E- oder 4-Alkenylgruppe oder Alkoxyalkylgruppe R³, der sich zwischen dem Ring C und der Doppelbindung bzw. dem Sauerstoffatom befindet, stets geradkettig; der restliche (endstellige) Teil dieser Alkenyl- oder Alkoxyalkylgruppe kann geradkettig oder verzweigt sein. Unter "Halogen" ist Fluor, Chlor, Brom oder Jod zu verstehen.

Sowohl die Stellung des Stickstoffatoms oder der Stickstoffatome im Pyridin-2,5-diylring bzw. Pyrimidin-2,5-diylring (Ring A oder B) als auch die Stellung des Sauerstoffatoms des trans-1,3-Dioxan-2,5-diylrings (Ring C) weist nur in die Richtung der halogenierten Phenylgruppe 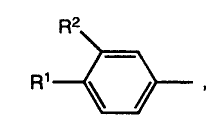 d.h. ein allfälliger solcher Ring ist in der obigen Formel I wie folgt angeordnet:

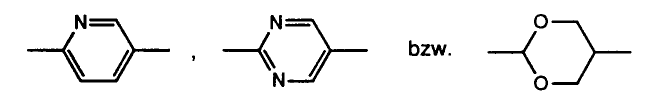

Unter dem in der Definition von Z¹ und Z² erscheinenden Ausdruck "gesättiger Ring" ist der trans-1,4-Cyclohexylenring (Ring A und/oder Ring B) zu verstehen. Der in der Massgabe (a) erscheinende Ausdruck "(hetero)aromatisch" bezieht sich auf den Ring B, wenn dieser unsubstituiertes oder mit Halogen und/oder Cyano und/oder Methyl mono- oder disubstituiertes 1,4-Phenylen; Pyridin-2.5-diyl; oder Pyrimidin-2,5-diyl bedeutet, d.h. verschieden von trans-1,4-Cyclohexylen ist.

Der Ring A ist vorzugsweise trans-1,4-Cyclohexylen oder gegebenenfalls substituiertes 1,4-Phenylen (wie dies oben näher beschrieben ist) und der Ring B vorzugsweise trans-1,4-Cyclohexylen, gefolgt von gegebenenfalls substituiertem 1,4-Phenylen. Vorzugsweise bedeuten Z¹ eine einfache Bindung, Z² und Z³ nicht beide Aethylen und R³ Vinyl, 1E-Propenyl, 1E- oder 3-Butenyl, 1E-, , 3E- oder 4-Pentenyl, 1E- oder 3E-Hexenyl oder 1E- oder 3E-Heptenyl. Falls der Ring A oder B gegebenenfalls substituiertes 1,4-Phenylen bedeutet, ist dies jeweils vorzugsweise unsubstituiertes 1,4-Phenylen.

Die Formel I umfasst vorzugsweise die Verbindungen der folgenden allgemeinen Formeln:

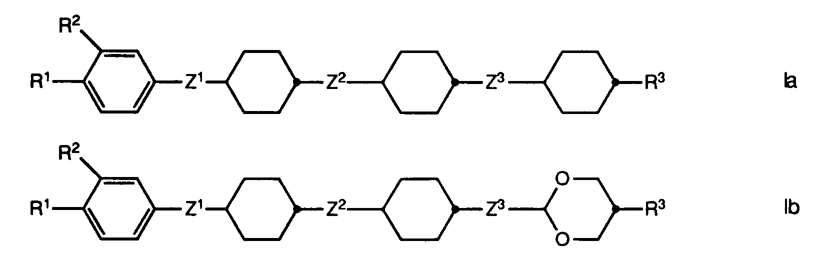

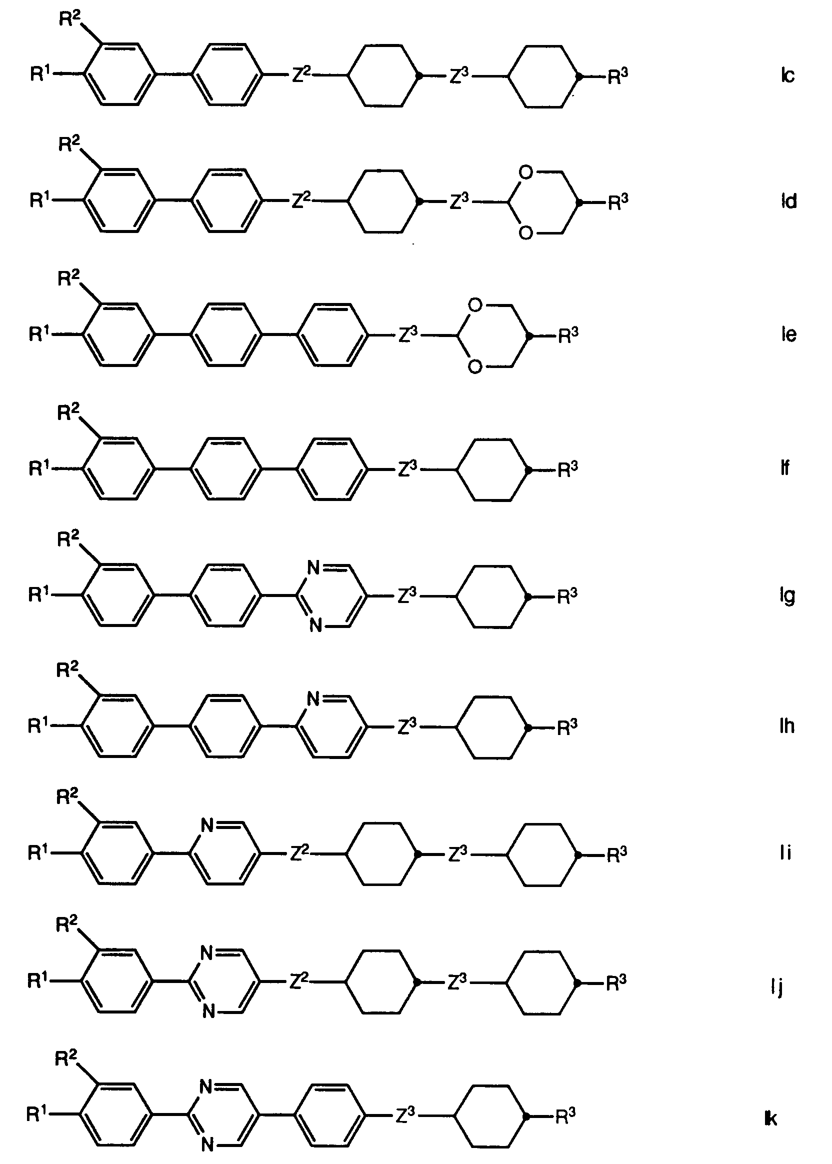

Einzelne Vertreter der Verbindungen der Formel I sind:
trans-5-(1E-Butenyl)-2-{trans-4'-(4-chlorphenyl)-[1,1'-bicyclohexyl]-trans-4-yl}-1,3-dioxan,
trans-5-(1E-Pentenyl)-2-{trans-4'-(4-chlorphenyl)-[1,1'-bicyclohexyl]-trans-4-yl}-1,3-dioxan,
trans-5-(4-Pentenyl)-2-{trans-4'-(4-chlorphenyl)-[1,1'-bicyclohexyl]-trans-4-yl}-1,3-dioxan,
trans-5-(3-Methoxypropyl)-2-{trans-4'-(4-chlorphenyl)-[1,1'-bicyclohexyl]-trans-4-yl}-1,3-dioxan,
trans-5-(1E-Propenyl)-2-{trans-4'-(4-chlor-3-fluorphenyl)-[1,1'-bicyclohexyl]-trans-4-yl}-1,3-dioxan,
trans-5-(3-Butenyl)-2-{trans-4'-(4-chlor-3-fluorphenyl)-[1,1'-bicyclohexyl]-trans-4-yl}-1,3-dioxan,
trans-5-(3-Methoxypropyl)-2-{trans-4'-(4-chlor-3-fluorphenyl)-[1,1'-bicyclohexyl]-trans-4-yl}-1,3-dioxan,
trans-5-(1E-Propenyl)-2-{trans-4'-(3,4-difluorphenyl)-[1,1'-bicyclohexyl]-trans-4-yl}-1,3-dioxan,
trans-5-(1E-Butenyl)-2-{trans-4'-(3,4-difluorphenyl)-[1,1'-bicyclohexyl]-trans-4-yl}-1,3-dioxan,
trans-5-(1E-Pentenyl)-2-{trans-4'-(3,4-difluorphenyl)-[1,1'-bicyclohexyl]-trans-4-yl}-1,3-dioxan,
trans-5-(3-Butenyl)-2-{trans-4'-(3,4-difluorphenyl)-[1,1'-bicyclohexyl]-trans-4-yl}-1,3-dioxan,
trans-5-(3-Methoxypropyl)-2-{trans-4'-(3,4-difluorphenyl)-[1,1'-bicyclohexyl]-trans-4-yl}-1,3-dioxan,
trans-5-(1E-Propenyl)-2-{trans-4'-(4-fluorphenyl)-[1,1'-bicyclohexyl]-trans-4-yl}-1,3-dioxan,
trans-5-(3-Methoxypropyl)-2-{trans-4'-(4-fluorphenyl)-[1,1'-bicyclohexyl]-trans-4-yl}-1,3-dioxan,
trans-5-(1E-Pentenyl)-2-{trans-4'-(4-chlorphenäthyl)-[1,1'-bicyclohexyl]-trans-4-yl}-1,3-dioxan,
trans-5-(4-Pentenyl)-2-{trans-4'-(4-chlorphenäthyl)-[1,1'-bicyclohexyl]-trans-4-yl}-1,3-dioxan,
trans-5-(3-Methoxypropyl)-2-{trans-4'-(4-chlorphenäthyl)-[1,1'-bicyclohexyl]-trans-4-yl}-1,3-dioxan,
trans-5-(1E-Propenyl)-2-{trans-4'-(4-chlor-3-fluorphenäthyl)-[1,1'-bicyclohexyl]-trans-4-yl}-1,3-dioxan,
trans-5-(3-Butenyl)-2-{trans-4'-(4-chlor-3-fluorphenäthyl)-[1,1'-bicyclohexyl]-trans-4-yl}-1,3-dioxan,
trans-5-(1E-Propenyl)-2-{trans-4'-(3,4-difluorphenäthyl)-[1,1'-bicyclohexyl]-trans-4-yl}-1,3-dioxan,
trans-5-(1E-Butenyl)-2-{trans-4'-(3,4-difluorphenäthyl)-[1,1'-bicyclohexyl]-trans-4-yl}-1,3-dioxan,
trans-5-(1E-Pentenyl)-2-{trans-4'-(3,4-difluorphenäthyl)-[1,1'-bicyclohexyl]-trans-4-yl}-1,3-dioxan,
trans-5-(3-Butenyl)-2-{trans-4'-(3,4-difluorphenäthyl)-[1,1'-bicyclohexyl]-trans-4-yl}-1,3-dioxan,
trans-5-(3-Methoxypropyl)-2-{trans-4'-(3,4-difluorphenäthyl)-[1,1'-bicyclohexyl]-trans-4-yl}-1,3-dioxan,
trans-5-(3-Methoxypropyl)-2-{trans-4'-(4-fluorphenäthyl)-[1,1'-bicyclohexyl]-trans-4-yl}-1,3-dioxan,
trans-5-(1E-Propenyl)-2-[2-{trans-4'-(4-chlorphenyl)-[1,1'-bicyclohexyl]-trans-4-yl}-äthyl]-1,3-dioxan,
trans-5-(3-Butenyl)-2-[2-{trans-4'-(4-chlorphenyl)-[1,1'-bicyclohexyl]-trans-4-yl}-äthyl]-1,3-dioxan,
trans-5-(3-Butenyl)-2-[2-{trans-4'-(3,4-difluorphenyl)-[1,1'-bicyclohexyl]-trans-4-yl}-äthyl]-1,3-dioxan,
trans-5-(3-Methoxypropyl)-2-[2-{trans-4'-(4-fluorphenyl)-[1,1'-bicyclohexyl]-trans-4-yl}-äthyl]-1,3-dioxan,
trans-5-(1E-Propenyl)-2-[2-{trans-4'-(4-chlorphenäthyl)-[1,1-bicyclohexyl]-trans-4-yl}-äthyl]-1,3-dioxan,
trans-5-(1E-Propenyl)-2-[trans-4-(4'-chlor-4-biphenylyl)-cyclohexyl]-1,3-dioxan,
trans-5-(1E-Pentenyl)-2-[trans-4-(4'-chlor-4-biphenylyl)-cyclohexyl]-1,3-dioxan,
trans-5-(3-Butenyl)-2-[trans-4-(4'-chlor-4-biphenylyl)-cyclohexyl]-1,3-dioxan,
trans-5-(4-Pentenyl)-2-[trans-4-(4'-chlor-4-biphenylyl)-cyclohexyl]-1,3-dioxan,
trans-5-(3-Methoxypropyl)-2-[trans-4-(4'-chlor-4-biphenylyl)-cyclohexyl]-1,3-dioxan,
trans-5-(1E-Propenyl)-2-[trans-4-(4'-chlor-3'-fluor-4-biphenylyl)-cyclohexyl]-1,3-dioxan,
trans-5-(3-Butenyl)-2-[trans-4-(4'-chlor-3'-fluor-4-biphenylyl)-cyclohexyl]-1,3-dioxan,
trans-5-(1E-Propenyl)-2-[trans-4-(3',4'-difluor-4-biphenylyl)-cyclohexyl]-1,3-dioxan,
trans-5-(3-Butenyl)-2-[trans-4-(3',4'-difluor-4-biphenylyl)-cyclohexyl]-1,3-dioxan,
trans-5-(3-Methoxypropyl)-2-[trans-4-(3',4'-difluor-4-biphenylyl)-cyclohexyl]-1,3-dioxan,
trans-5-(4-Pentenyl)-2-[trans-4-(4'-fluor-4-biphenylyl)-cyclohexyl]-1,3-dioxan,
trans-5-(3-Methoxypropyl)-2-[trans-4-(4'-fluor-4-biphenylyl)-cyclohexyl]-1,3-dioxan,
trans-5-(1E-Propenyl)-2-{trans-4-[2-(4'-chlor-4-biphenylyl)-äthyl]-cyclohexyl}-1,3-dioxan,
trans-5-(3-Butenyl)-2-{trans-4-[2-(4'-chlor-4-biphenylyl)-äthyl]-cyclohexyl}-1,3-dioxan,
trans-5-(1E-Propenyl)-2-{trans-4-[2-(3',4'-difluor-4-biphenylyl)-äthyl]-cyclohexyl}-1,3-dioxan,
trans-5-(3-Butenyl)-2-{trans-4-[2-(3',4'-difluor-4-biphenylyl)-äthyl]-cyclohexyl}-1,3-dioxan,
trans-5-(3-Methoxypropyl)-2-{trans-4-[2-(4'-fluor-4-biphenylyl)-äthyl]-cyclohexyl}-1,3-dioxan,
trans-5-(3-Butenyl)-2-(4''-chlor-p-terphenyl-4-yl)-1,3-dioxan,
trans-5-(3-Methoxypropyl)-2-(4''-fluor-p-terphenyl-4-yl)-1,3-dioxan,
trans-5-(3-Butenyl)-2-[2-(4''-fluor-p-terphenyl-4-yl)-äthyl]-1,3-dioxan,
trans-5-(trans-4-Vinylcyclohexyl)-2-[trans-4-(4-chlorphenyl)-cyclohexyl]-1,3-dioxan,
trans-5-[trans-4-(3-Butenyl)-cyclohexyl]-2-[trans-4-(4-chlorphenyl)-cyclohexyl]-1,3-dioxan,
trans-5-[trans-4-(3-Methoxypropyl)-cyclohexyl]-2-[trans-4-(4-chlorphenyl)-cyclohexyl]-1,3-dioxan,
trans-5-(trans-4-Vinylcyclohexyl)-2 [trans-4-(3,4-difluorphenyl)-cyclohexyl]-1,3-dioxan,
trans-5-[trans-4-(3-Butenyl)-cyclohexyl]-2-[trans-4-(3,4-difluorphenyl)-cyclohexyl]-1,3-dioxan,
trans-5-[trans-4-(3-Methoxypropyl)-cyclohexyl]-2-[trans-4-(4-fluorphenyl)-cyclohexyl]-1,3-dioxan,
trans-5-(trans-4-Vinylcyclohexyl)-2-{2-[trans-4-(4-chlorphenyl)-cyclohexyl]-äthyl}-1,3-dioxan,
trans-5-[trans-4-(3-Butenyl)-cyclohexyl]-2-{2-[trans-4-(4-chlorphenyl)-cyclohexyl]-äthyl}-1,3-dioxan,
trans-5-[trans-4-(3-Butenyl)-cyclohexyl]-2-{2-[trans-4-(3,4-difluorphenyl)-cyclohexyl]-äthyl}-1,3-dioxan,
trans-5-(trans-4-Vinylcyclohexyl)-2-(4'-chlor-4-biphenylyl)-1,3-dioxan,
trans-5-[trans-4-(3-Butenyl)-cyclohexyl]-2-(4'-chlor-4-biphenylyl)-1,3-dioxan,
trans-5-[trans-4-(3-Butenyl)-cyclohexyl]-2-(3',4'-difluor-4-biphenylyl)-1,3-dioxan,
trans-5-[trans-4-(3-Methoxypropyl)-cyclohexyl]-2-(4'-fluor-4-biphenylyl)-1,3-dioxan,
trans-5-[trans-4-(3-Butenyl)-cyclohexyl]-2-[2-(4'-chlor-4-biphenylyl)-äthyl]-1,3-dioxan,
4-[trans-4'-Vinyl-(1,1'-bicyclohexyl)-trans-4-yl]-3',4'-difluorbiphenyl,
4-{trans-4'-(1E-Pentenyl)-[1,1'-bicyclohexyl]-trans-4-yl}-3',4'-difluorbiphenyl,
4-{trans-4'-(3-Butenyl)-[1,1'-bicyclohexyl]-trans-4-yl}-3',4'-difluorbiphenyl,
4-{trans-4'-(3E-Pentenyl)-[1,1'-bicyclohexyl]-trans-4-yl}-3',4'-difluorbiphenyl,
4-{trans-4'-(4-Pentenyl)-[1,1'-bicyclohexyl]-trans-4-yl}-4'-fluorbiphenyl,
4-[trans-4'-Vinyl-(1,1'-bicyclohexyl)-trans-4-yl]-4'-chlorbiphenyl,
4-{trans-4'-(1E-Propenyl)-[1,1'-bicyclohexyl]-trans-4-yl}-4'-chlorbiphenyl,
4-{trans-4'-(3-Butenyl)-[1,1'-bicyclohexyl]-trans-4-yl}-4'-chlorbiphenyl,
4-{trans-4'-(3E-Pentenyl)-[1,1'-bicyclohexyl]-trans-4-yl}-4'-chlorbiphenyl,
4-[trans-4'-Vinyl-(1',1'-bicyclohexyl)-trans-4-yl]-4'-chlor-3'-fluorbiphenyl,
4-{trans-4'-(3-Butenyl)-[1,1'-bicyclohexyl]-trans-4-yl}-4'-chlor-3'-fluorbiphenyl,
4-{2-[trans-4'-Vinyl-(1,1'-bicyclohexyl)-trans-4-yl]-äthyl}-3',4'-difluorbiphenyl,
4-[2-{trans-4'-(1E-Propenyl)-[1,1'-bicyclohexyl]-trans-4-yl}-äthyl]-3',4'-difluorbiphenyl,
4-[2-{trans-4'-(3-Butenyl)-[1,1'-bicyclohexyl]-trans-4-yl}-äthyl]-3',4'-difluorbiphenyl,
4-{2-[trans-4'-Vinyl-(1,1'-bicyclohexyl)-trans-4-yl]-äthyl}-4'-chlorbiphenyl,
4-[2-{trans-4'-(3-Butenyl)-[1,1'-bicyclohexyl]-trans-4-yl}-äthyl]-4'-chlorbiphenyl,
4-{trans-4-[2-(trans-4-Vinylcyclohexyl)-äthyl]-cyclohexyl}-3',4'-difluorbiphenyl,
4-[trans-4-{2-[trans-4-(3-Butenyl)-cyclohexyl]-äthyl}-cyclohexyl]-4'-chlorbiphenyl,
trans-4-[2-(trans-4-Vinylcyclohexyl)-äthyl]-trans-4'-(3,4-difluorphenyl)-1,1'-bicyclohexyl,
trans-4-{2-[trans-4-(1E-Propenyl)-cyclohexyl]-äthyl}-trans-4'-(3,4-difluorphenyl)-1,1'-bicyclohexyl,
trans-4-{2-[trans-4-(3-Butenyl)-cyclohexyl]-äthyl}-trans-4'-(3,4-difluorphenyl)-1,1'-bicyclohexyl,
trans-4-{2-[trans-4-(4-Pentenyl)-cyclohexyl]-äthyl}-trans-4'-(4-fluorphenyl)-1,1-bicyclohexyl,
trans-4-[2-(trans-4-Vinylcyclohexyl)-äthyl]-trans-4'-(4-chlor-3-fluorphenyl)-1,1'-bicyclohexyl,
trans-4-[2-(trans-4-Vinylcyclohexyl)-äthyl]-trans-4'-(4-chlorphenyl)-1,1'-bicyclohexyl,
trans-4-{2-[trans-4-(1E-Propenyl)-cyclohexyl]-äthyl}-trans-4'-(4-chlor-3-fluorphenyl)-1,1'-bicyclohexyl,
trans-4-{2-[trans-4-(3-Butenyl)-cyclohexyl]-äthyl]-trans-4'-(4-chlor-3-fluorphenyl)-1,1'-bicyclohexyl,
trans-4-Vinyl-trans-4'-{2-[trans-4-(3,4-difluorphenyl)-cyclohexyl]-äthyl}-1,1'-bicyclohexyl,
trans-4-(1E-Propenyl)-trans-4'-{2-[trans-4-(3,4-difluorphenyl)-cyclohexyl]-äthyl}-1,1'-bicyclohexyl,
trans-4-Vinyl-trans-4'-{2-[trans-4-(4-chlorphenyl)-cyclohexyl]-äthyl}-1,1'-bicyclohexyl,
trans-4-(1E-Propenyl)-trans-4'-{2-[trans-4-(4-chlorphenyl)-cyclohexyl]-äthyl}-1,1'-bicyclohexyl,
trans-4-(3-Butenyl)-trans-4'-{2-[trans-4-(4-chlorphenyl)-cyclohexyl]-äthyl}-1,1'-bicyclohexyl,
4-(trans-4-Vinylcyclohexyl)-3'',4''-difluor-p-terphenyl,
4-[trans-4-(3-Butenyl)-cyclohexyl]-4''-chlor-p-terphenyl,
4-[2-(trans-4-Vinylcyclohexyl)-äthyl]-3'',4''-difluor-p-terphenyl,
4-{2-[trans-4-(3-Butenyl)-cyclohexyl]-äthyl}-4''-chlor-p-terphenyl,
5-[trans-4'-Vinyl-(1,1'-bicyclohexyl)-trans-4-yl]-2-(3,4-difluorphenyl)-pyridin,
5-[trans-4'-Vinyl-(1,1'-bicyclohexyl)-trans-4-yl]-2-(4-chlorphenyl)-pyridin,
5-{2-[trans-4'-Vinyl-(1,1'-bicyclohexyl)-trans-4-yl]-äthyl}-2-(3,4-difluorphenyl)-pyridin,
5-[2-{trans-4'-(1E-Propenyl)-[1,1'-bicyclohexyl]-trans-4-yl}-äthyl]-2-(3,4-difluorphenyl)-pyridin,
5-[2-{trans-4'-(3-Butenyl)-[1,1'-bicyclohexyl]-trans-4-yl}-äthyl]-2-(3,4-difluorphenyl)-pyridin,
5-[2-{trans-4'-(4-Pentenyl)-[1,1'-bicyclohexyl]-trans-4-yl}-äthyl]-2-(4-fluorphenyl)-pyridin,
5-{2-[trans-4'-Vinyl-(1,1'-bicyclohexyl)-trans-4-yl]-äthyl}-2-(4-chlor-3-fluorphenyl)-pyridin,
5-{2-[trans-4'-Vinyl-(1,1'-bicyclohexyl)-trans-4-yl]-äthyl}-2-(4-chlorphenyl)-pyridin,
5-[2-{trans-4'-(1E-Propenyl)-[1,1'-bicyclohexyl]-trans-4-yl}-äthyl]-2-(4-chlorphenyl)-pyridin,
5-[2-{trans-4'-(3-Butenyl)-[1,1'-bicyclohexyl]-trans-4-yl}-äthyl]-2-(4-chlorphenyl)-pyridin,
4-{trans-4'-(3-Methoxypropyl)-[1,1'-bicyclohexyl]-trans-4-yl}-4'-fluorbiphenyl,
4-{trans-4'-(3-Methoxypropyl)-[1,1'-bicyclohexyl]-trans-4-yl}-4'-chlor-3'-fluorbiphenyl,
4-{trans-4'-(3-Methoxypropyl)-[1,1'-bicyclohexyl]-trans-4-yl}-4'-chlorbiphenyl,
4-[2-{trans-4'-(3-Methoxypropyl)-[1,1'-bicyclohexyl]-trans-4-yl}-äthyl]-3',4'-difluorbiphenyl,
4-[2-{trans-4'-(3-Methoxypropyl)-[1,1'-bicyclohexyl]-trans-4-yl}-äthyl]-4'-fluorbiphenyl,
4-[2-{trans-4'-(3-Methoxypropyl)-[1,1'-bicyclohexyl]-trans-4-yl}-äthyl]-4'-chlor-3'-fluorbiphenyl,
trans-4-{2-[trans-4-(3-Methoxypropyl)-cyclohexyl]-äthyl}-trans-4'-(4-fluorphenyl)-1,1'-bicyclohexyl,
trans-4-{2-[trans-4-(3-Methoxypropyl)-cyclohexyl]-äthyl}-trans-4'-(4-chlorphenyl)-1,1'-bicyclohexyl,
5-[2-{trans-4'-(3-Methoxypropyl)-[1,1'-bicyclohexyl]-trans-4-yl}-äthyl]-2-(3,4-difluorphenyl)-pyridin,
5-[2-{trans-4'-(3-Methoxypropyl)-[1,1'-bicyclohexyl]-trans-4-yl}-äthyl]-2-(4-fluorphenyl)-pyridin,
5-[2-{trans-4'-(3-Methoxypropyl)-[1,1'-bicyclohexyl]-trans-4-yl}-äthyl]-2-(4-chlorphenyl)-pyridin,
5-{trans-4'-Vinyl-[1,1'-bicyclohexyl]-trans-4-yl}-2-(4-chlorphenyl)-pyrimidin,
5-{trans-4'-(3-Butenyl)-[1,1'-bicyclohexyl]-trans-4-yl}-2-(4-chlorphenyl)-pyrimidin,
5-{trans-4'-(3-Methoxypropyl)-[1,1'-bicyclohexyl]-trans-4-yl}-2-(4-chlorphenyl)-pyrimidin,
5-{trans-4'-(4-Pentenyl)-[1,1'-bicyclohexyl]-trans-4-yl}-2-(4-fluorphenyl)-pyrimidin,
5-{trans-4'-(3-Methoxypropyl)-[1,1'-bicyclohexyl]-trans-4-yl}-2-(4-fluorphenyl)-pyrimidin,
5-{trans-4'-(1E-Propenyl)-[1,1'-bicyclohexyl]-trans-4-yl}-2-(3,4-difluorphenyl)-pyrimidin,
5-[2-{trans-4'-Vinyl-[1,1'-bicyclohexyl]-trans-4-yl}-äthyl]-2-(4-chlorphenyl)-pyrimidin,
5-[2-{trans-4'-(3-Butenyl)-[1,1'-bicyclohexyl]-trans-4-yl}-äthyl]-2-(4-chlorphenyl)-pyrimidin,
5-[2-{trans-4'-(3-Methoxypropyl)-[1,1'-bicyclohexyl]-trans-4-yl]-äthyl]-2-(4-fluorphenyl)-pyrimidin und
5-[2-{trans-4'-(1E-Propenyl)-[1,1'-bicyclohexyl]-trans-4-yl}-äthyl]-2-(3,4-difluorphenyl)-pyrimidin.

Die Verbindungen der Formel I können nach an sich bekannten Methoden hergestellt werden, z.B. gemäss den nachfolgenden Reaktionsschemata 1-7, von denen die Schemata 1a, 1b und 2 die Herstellung der Endprodukte I und Schemata 3a, 3b und 4-7 diejenige der wichtigen z.T. neuen Zwischenprodukte bzw. Ausgangsmaterialien aus bekannten oder auf an sich bekannte Weise herstellbaren Verbindungen darstellen. Die angewendeten Methoden, z.B. die Wittig-Reaktion, die Grignard-Reaktion, Hydrolysen, Reduktionen, Halogenierungen usw. sind reichlich in der Fachliteratur beschrieben und sind jedem Fachmann geläufig.

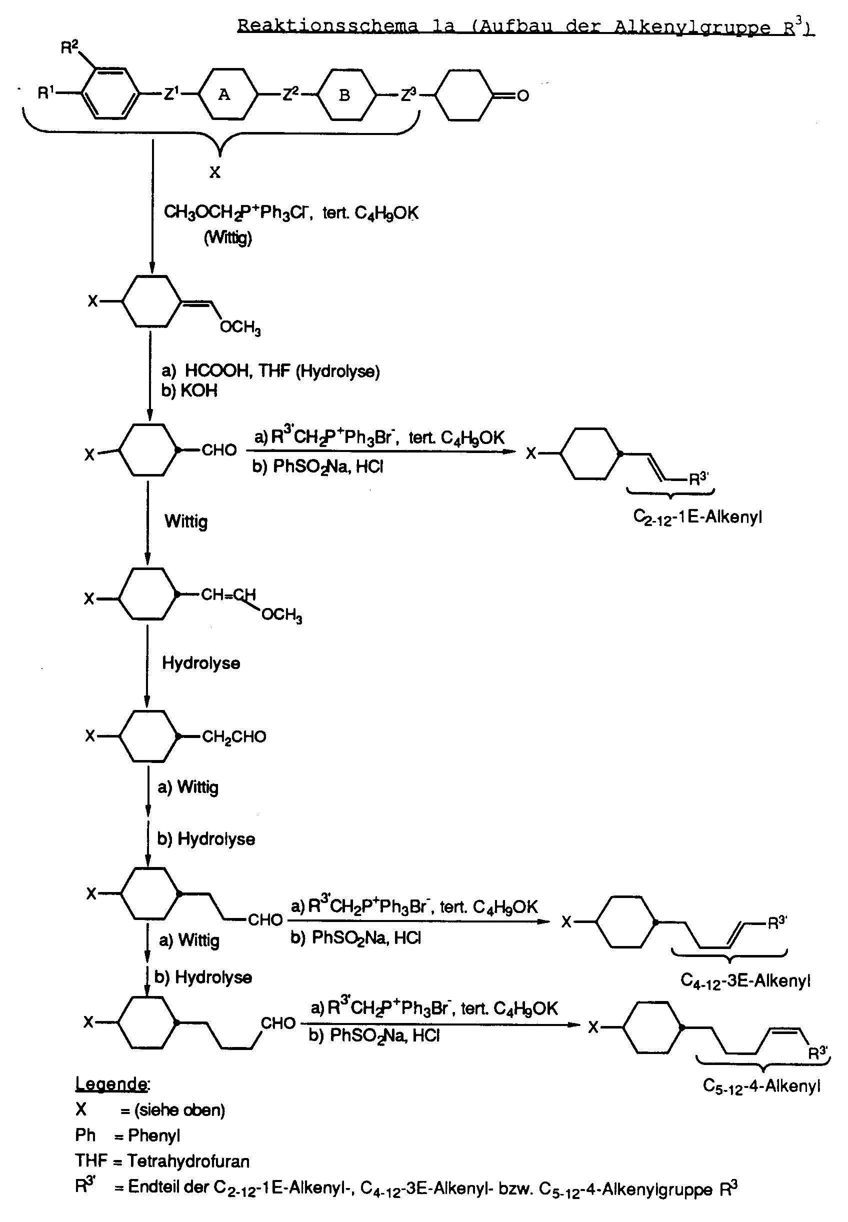

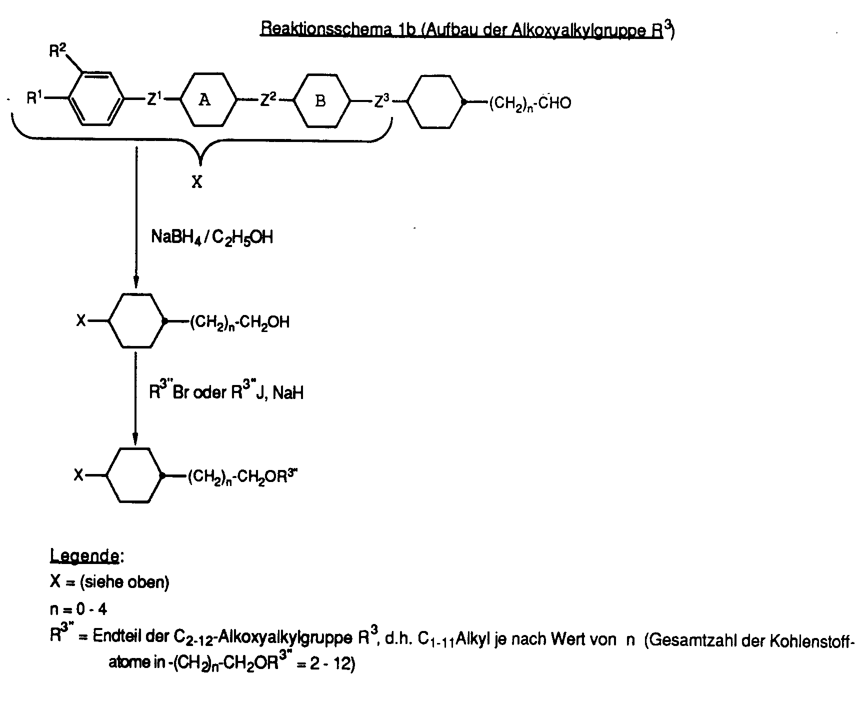

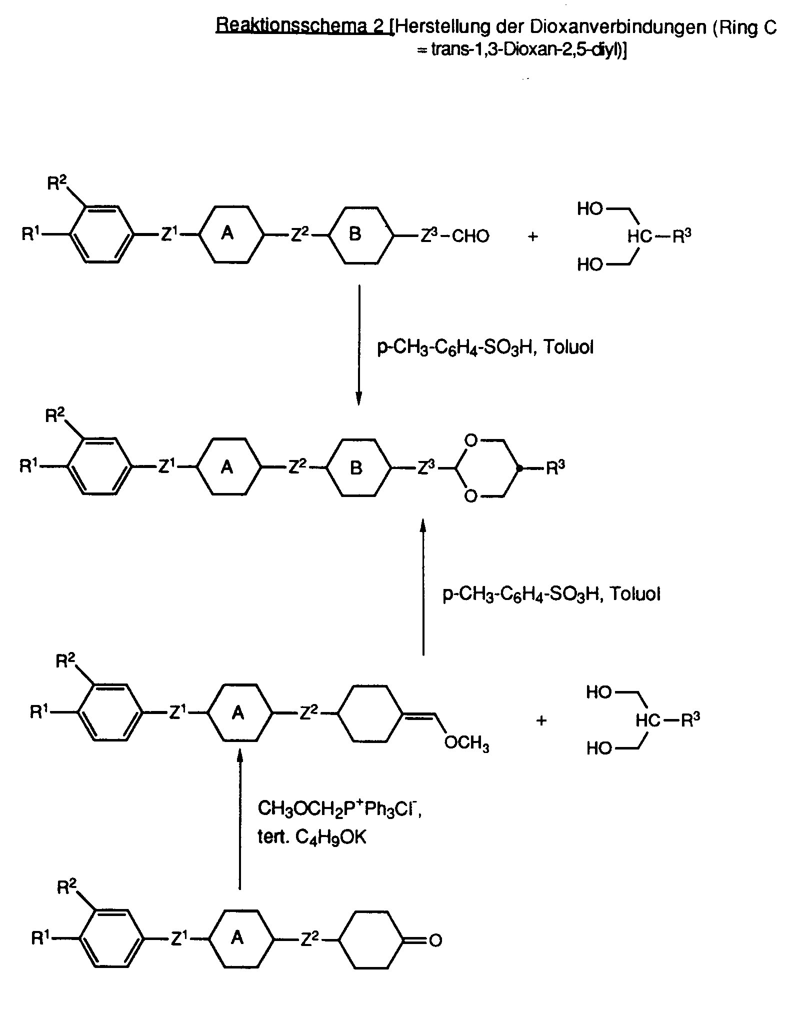

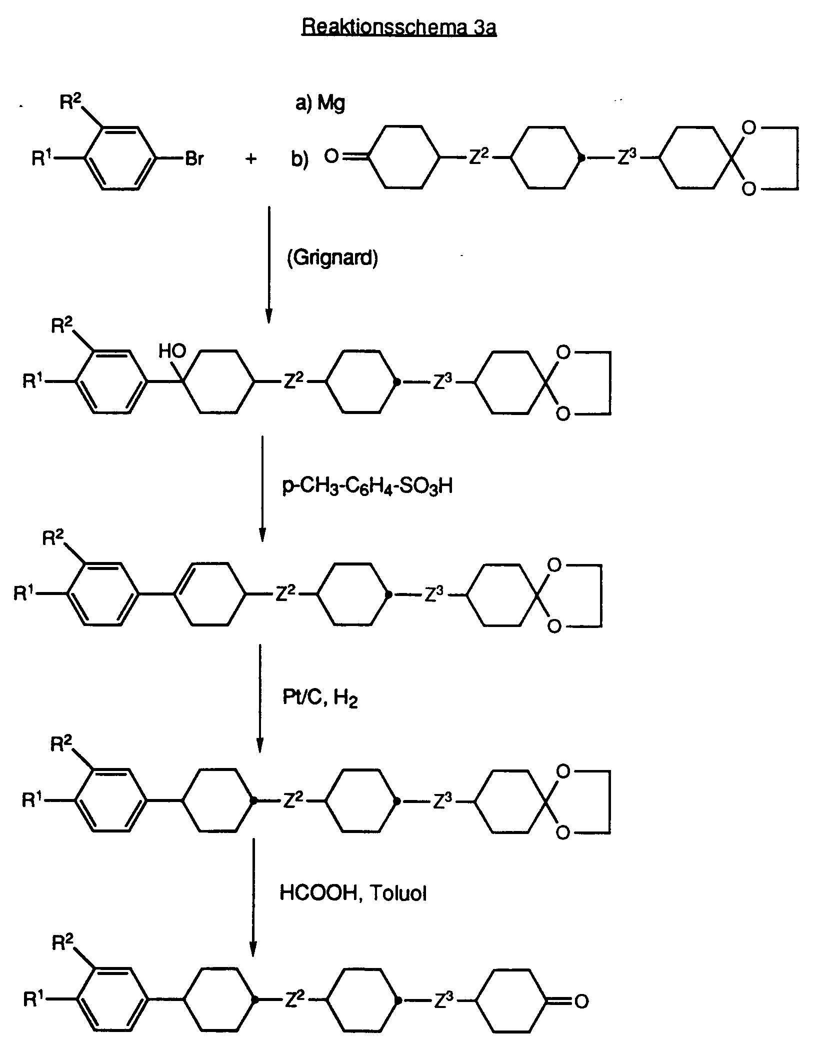

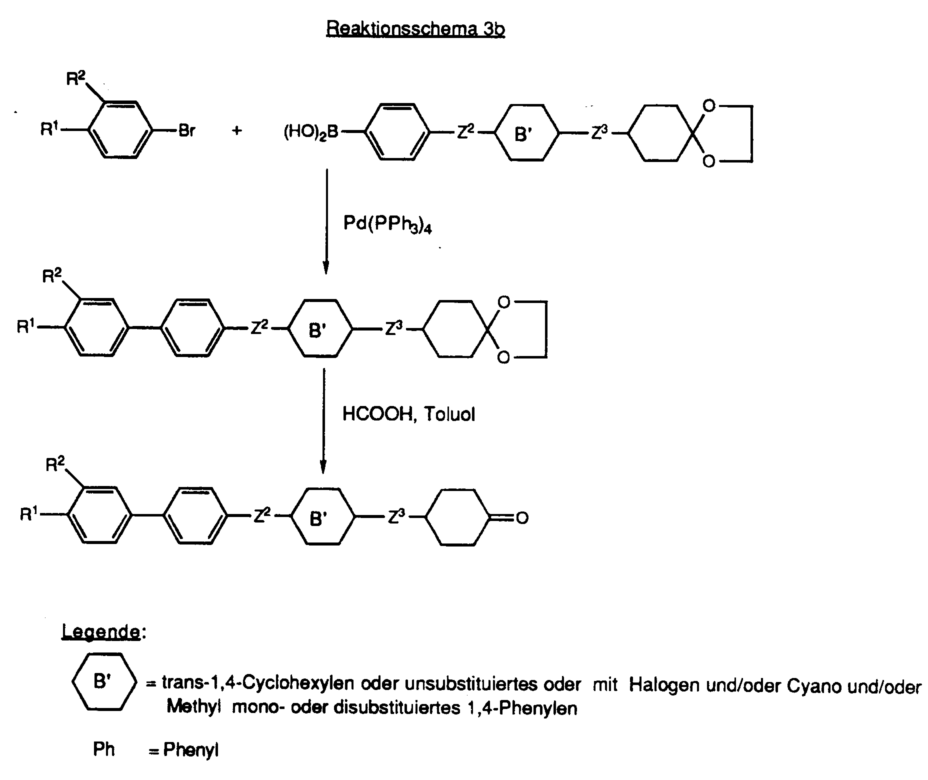

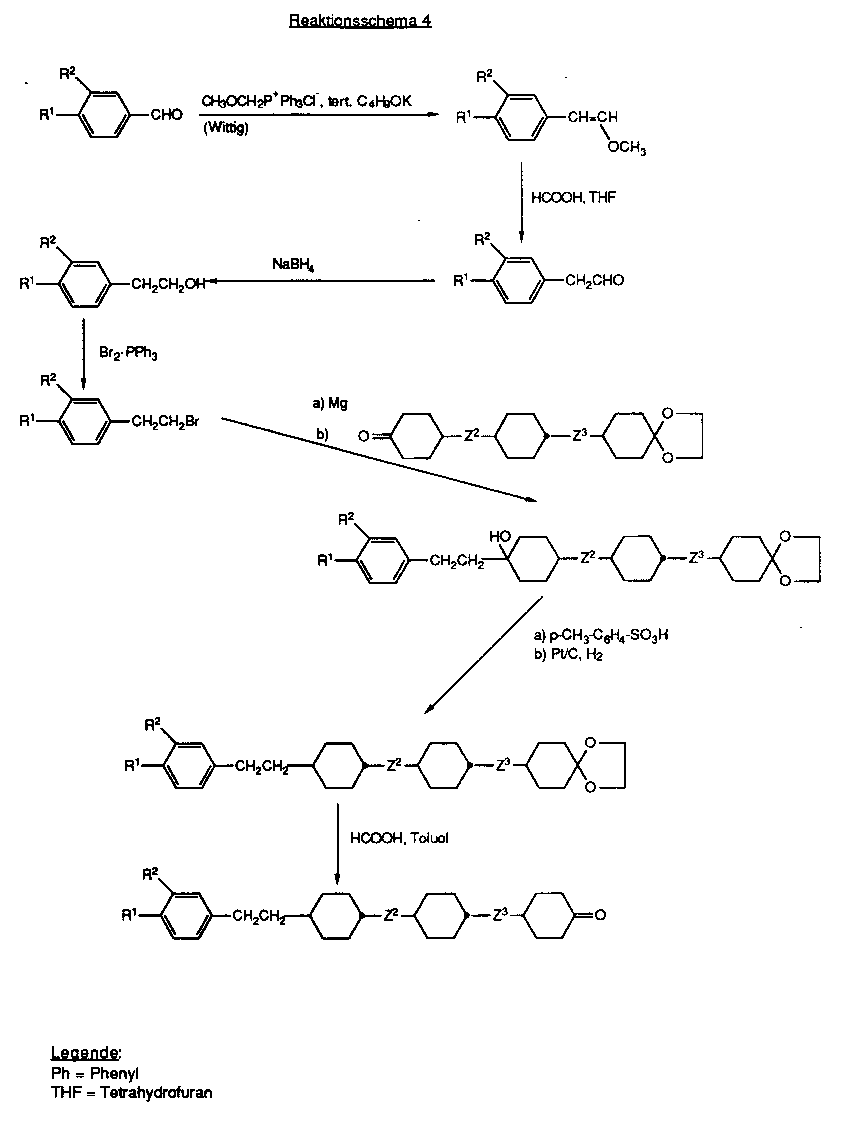

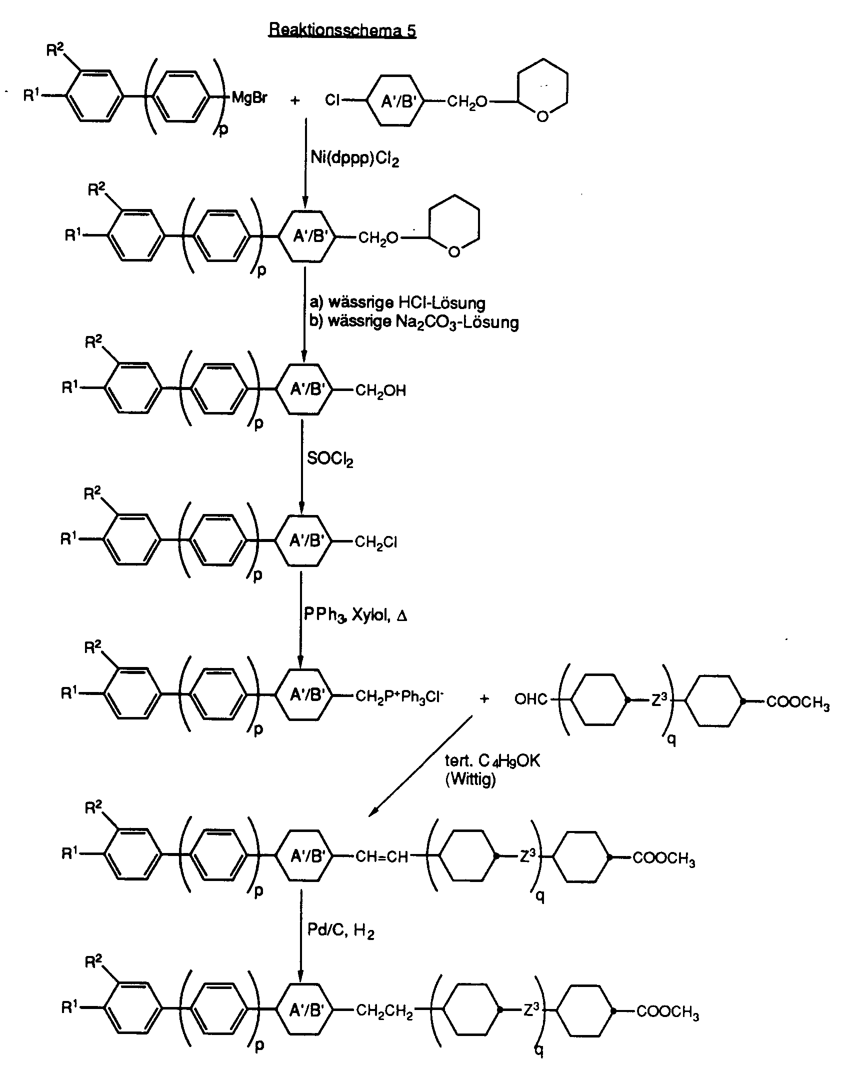

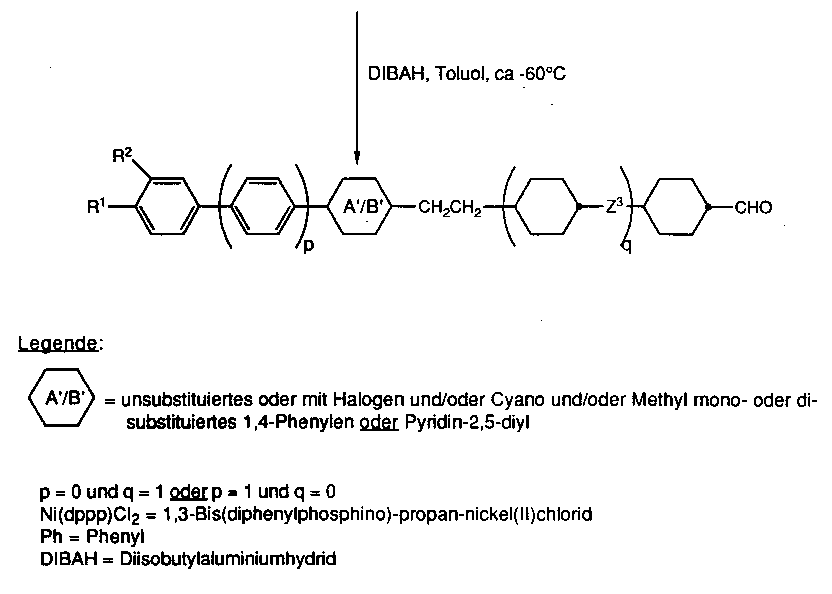

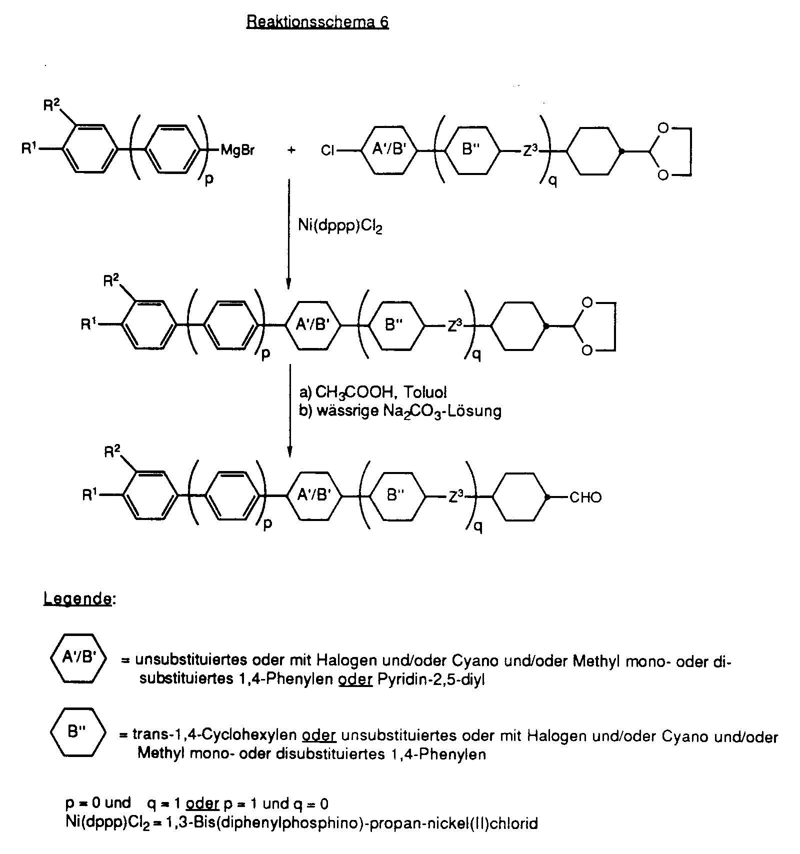

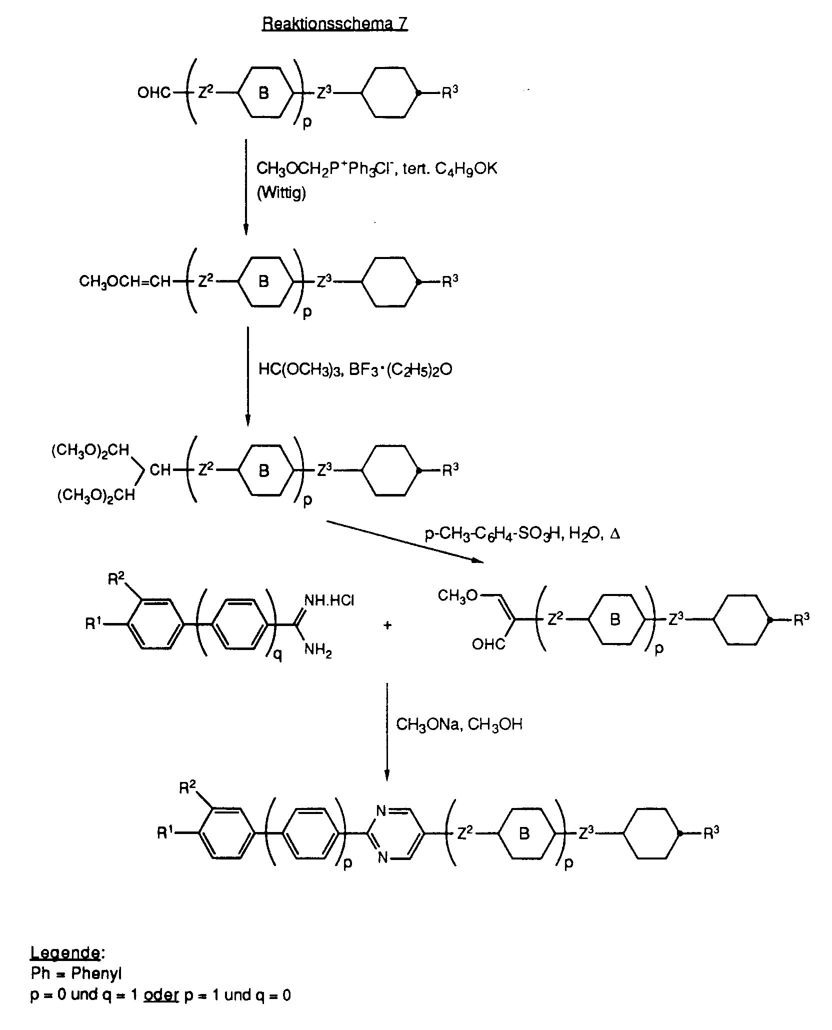

Wenn R³ verschieden von Vinyl oder C₂₋₁₂-Alkoxyalkyl ist, werden in der Regel E/Z-Gemische erhalten, welche nach an sich bekannten Methoden, z.B. chromatographisch an mit Silbernitrat imprägniertem Kieselgel, getrennt werden können. Ferner können gewünschtenfalls die E/Z-Gemische bzw. die Z-Isomere durch Aequilibrierung mit Sulfinsäuren, z.B. Benzolsulfinsäure oder p-Toluolsulfinsäure, überwiegend in die E-Form übergeführt werden.

Die Verbindungen der Formel I können in Form von Gemischen untereinander und mit anderen Flüssigkristallkomponenten verwendet werden, wie z.B. mit Substanzen aus den Klassen der Azoxybenzole, Phenylbenzoate, Cyclohexancarbonsäure-phenylester, Cyclohexancarbonsäure-cyclohexylester, Biphenyle, Tolane, Phenylcyclohexane, Cyclohexylcyclohexane, Phenylpyrimidine, Phenylpyridine, Cyclohexylpyrimidine, Cyclohexylpyridine, Phenyldioxane, 2-Cyclohexyl-1-phenyläthane, Terphenyle, Cyclohexylbiphenyle, Bicyclohexylphenyle, Cyclohexylphenylpyrimidine, Phenylcyclohexyldioxane und Dicyclohexylbiphenyle. Derartige Substanzen sind dem Fachmann bekannt, und viele davon sind zudem im Handel erhältlich.

Die erfindungsgemässen flüssigkristallinen Gemische enthalten mindestens zwei Komponenten, wovon mindestens eine Komponente eine Verbindung der Formel I ist. . Die Verbindungen der Formel I eignen sich insbesondere für nematische oder, sofern mindestens eine Komponente des Gemisches optisch aktiv ist, auch für cholesterische Gemische.

Aufgrund der guten Löslichkeit der Verbindungen der Formel I und aufgrund ihrer guten Mischbarkeit untereinander kann ihr Anteil in den erfindungsgemässen Gemischen relativ hoch sein. Im allgemeinen ist jedoch ein Anteil von etwa 1-50 Gew.-%, insbesondere etwa 2-30 Gew.-%, an Verbindungen der Formel I bevorzugt.

Die erfindungsgemässen Gemische enthalten neben einer oder mehreren Verbindungen der Formel I eine oder mehrere Verbindungen aus der Gruppe der Verbindungen der allgemeinen Formeln: 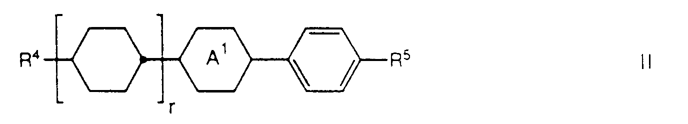 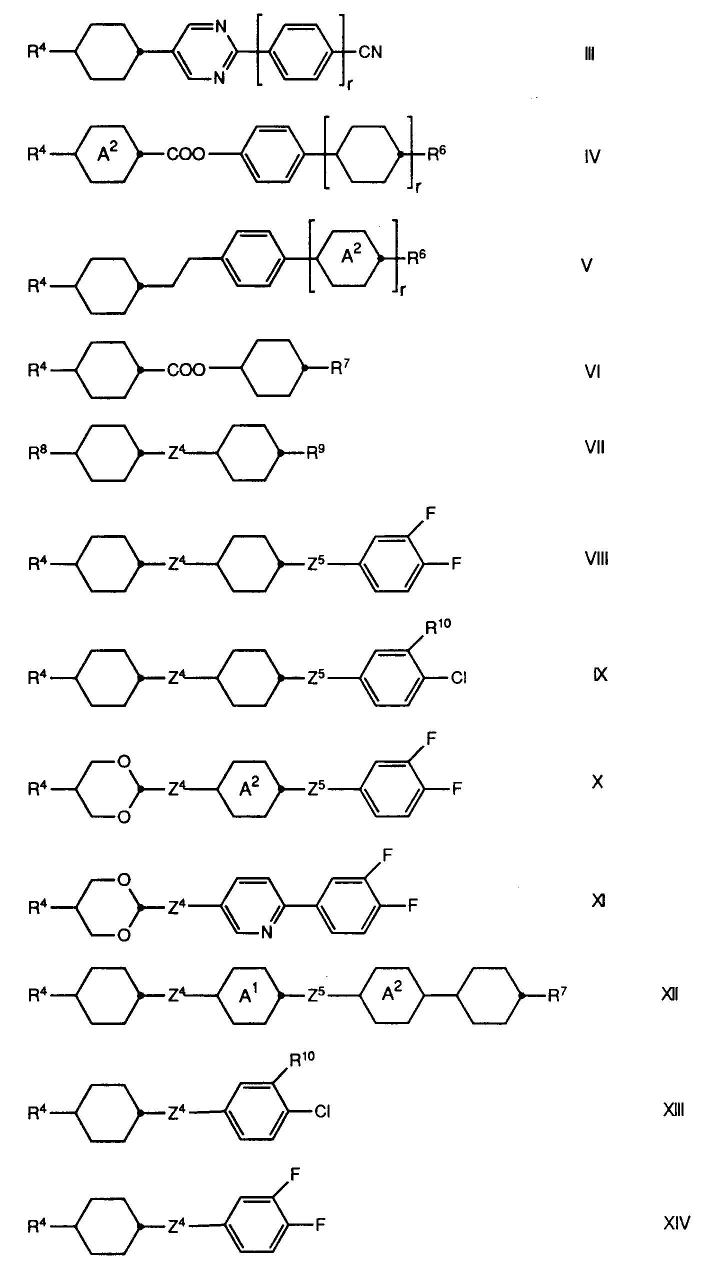 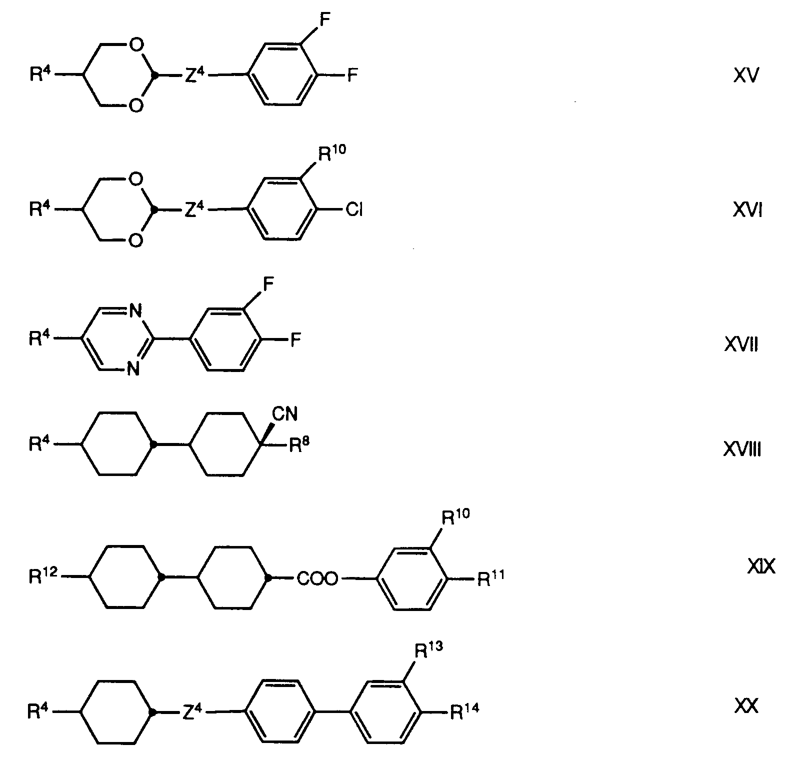 worin r für die Zahl 0 oder 1 steht; R⁴ und R⁷ unabhängig voneinander Alkyl, Alkoxyalkyl, 3E-Alkenyl, 4-Alkenyl oder an einem gesättigten Ring auch 1E-Alkenyl bedeutet; Ring A¹ 1,4-Phenylen, trans-1,4-Cyclohexylen, Pyridin-2,5-diyl, Pyrimidin-2,5-diyl oder trans-1,3-Dioxan-2,5-diyl darstellt; R⁵ Cyano, Isothiocyanato, Fluor, Alkyl, 3E-Alkenyl, 4-Alkenyl, Alkoxy, 2E-Alkenyloxy, 3-Alkenyloxy oder 1-Alkinyl bezeichnet; Ring A² 1,4-Phenylen oder trans-1,4-Cyclohexylen darstellt; R⁶ Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet; R⁸ Alkyl, 1E-Alkenyl, 3E-Alkenyl oder 4-Alkenyl bezeichnet; Z⁴ und Z⁵ jeweils eine einfache Bindung oder Aethylen darstellt, wobei zwei aromatische Ringe nur durch eine einfache Bindung gebunden sind; R⁹ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet; R¹⁰ Wasserstoff, Fluor oder Chlor bezeichnet; R¹¹ Fluor, Chlor oder Cyano bedeutet; R¹² Alkyl,
1E-Alkenyl, 3E-Alkenyl oder 4-Alkenyl darstellt; R¹³ Wasserstoff oder Fluor bedeutet; und R¹⁴ Fluor oder Chlor bezeichnet.

Der obige Ausdruck "gesättigter Ring" umfasst trans-1,4-Cyclohexylen und trans-1,3-Dioxan-2,5-diyl. Reste R⁴ bis R⁹ und R¹² besitzen vorzugsweise höchstens je 12 Kohlenstoffatome, besonders bevorzugt höchstens je 7 Kohlenstoffatome. Geradkettige Reste sind im allgemeinen bevorzugt.

Die Herstellung der flüssigkristallinen Gemische und der elektrooptischen Vorrichtungen kann in an sich bekannter Weise erfolgen.

Die Erfindung wird durch die folgenden Beispiele weiter veranschaulicht. In den Beispielen bedeutet C eine kristalline, N eine nematische und I die isotrope Phase.

### Beispiel 1

a) Eine Suspension von 5,7 g Methoxymethyltriphenylphosphoniumchlorid in 100 ml tert.Butylmethyläther wird bei 0°C unter Rühren und Stickstoffbegasung mit 1,8 g Kalium-tert.butylat versetzt. Die Suspension wird weitere 90 Minuten bei 10°C gerührt, dann bei 0°C innert 10 Minuten tropfenweise mit einer Lösung von 2,4 g trans-4'-(4-Chlorphenyl)-[1,1'-bicyclohexyl]-4-on in 50 ml tert.Butylmethyläther/Methylenchlorid (4:1) versetzt und noch 2 Stunden bei Raumtemperatur gerührt. Dann wird mit 100 ml Diäthyläther verdünnt, dreimal mit je 100 ml Wasser gewaschen, und die organische Phase mit wasserfreiem Magnesiumsulfat getrocknet, filtriert und unter vermindertem Druck eingedampft. Chromatographische Reinigung des Rückstandes (11 g) an Kieselgel mit n-Hexan/Aethylacetat liefert 2,5 g trans-4-(4-Chlorphenyl)-4'-(methoxymethyliden)-[1,1'-bicyclohexyl] als farblose Kristalle.
b) Eine Lösung von 2,6 trans-4-(4-Chlorphenyl)-4'-(methoxymethyliden)-[1,1'-bicyclohexyl], 1,1 g 2-(3-Butenyl)-propan-1,3-diol und 0,1 g p-Toluolsulfonsäure in 80 ml Toluol wird langsam auf 120°C (Badtemperatur) erwärmt und ca. ein Drittel des Toluolvolumens abdestilliert. Danach wird das Reaktionsgemisch bei Rückflusstemperatur erhitzt, nach 15 Stunden abgekühlt und auf eine 5%ige Lösung von wässrigem Natriumbicarbonat gegossen. Das wässrige Gemisch wird mit ca. 100 ml Diäthyläther versetzt und die abgetrennte organische Phase nochmals mit Natriumbicarbonatlösung gewaschen, über wasserfreiem Magnesiumsulfat getrocknet und unter vermindertem Druck eingedampft. Der Rückstand (3,4 g) wird hierauf an Kieselgel mit Toluol/Essigester (1:1) chromatographiert. Kristallisation aus n-Hexan ergibt 1,93 g trans-5-(3-Butenyl)-2-{trans-4'-(4-chlorphenyl)-[1,1'-bicyclohexyl]-trans-4-yl}-1,3-dioxan als farblose Kristalle, Smp. (C-S_{B}) 94°C; S_{B}-N 189,3°C; Klp. (N-I) >280°C.

In analoger Weise werden folgende Verbindungen I hergestellt:
trans-5-(1E-Propenyl)-2-{trans-4'-(4-chlorphenyl)-[1,1'-bicyclohexyl]-trans-4-yl}-1,3-dioxan, Smp. (C-S_{B}) 117,9°C; S_{B}-N 199°C; Klp. (N-I) >300°C.
trans-5-(1E-Propenyl)-2-{trans-4'-(4-chlorphenäthyl)-[1,1'-bicyclohexyl]-trans-4-yl}-1,3-dioxan, Smp. (C-S_{B}) 128,6°C; S_{B}-N 232°C; Klp. (N-I) 300°C.
trans-5-(3-Butenyl)-2-{trans-4'-(4-chlorphenäthyl)-[1,1'-bicyclohexyl]-trans-4-yl}-1,3-dioxan, Smp. (C-S_{B}) 75,4°C; S_{B}-N 240°C; Klp. (N-I) 262°C.
trans-5-(1E-Pentenyl)-2-{trans-4'-(4-fluorphenäthyl)-[1,1'-bicyclohexyl]-trans-4-yl}-1,3-dioxan, Smp. (C-S_{B}) 86,8°C; S_{B}-N 269°C, Klp. (N-I) 278°C.
trans-5-(4-Pentenyl)-2-{trans-4'-(4-fluorphenäthyl)-[1,1'-bicyclohexyl]-trans-4-yl}-1,3-dioxan, Smp. (C-S_{B}) ca. 40°C; Klp. (S_{B}-I) 235°C.
trans-5-(4-Pentenyl)-2-[2-{trans-4'-(4-fluorphenäthyl)-[1,1'-bicyclohexyl]-trans-4-yl}-äthyl]-1,3-dioxan, Smp. (C-S_{B}) 79°C; Klp. (S_{B}-I) 223°C.

### Beispiel 2

a) Zu 33 mg Magnesiumspänen in 0,5 ml trockenem Tetrahydrofuran wird 0,1 ml einer Lösung von ca. 10 µl Dibromäthan in 1 ml Tetrahydrofuran zugesetzt. Dann wird eine Lösung von 420 mg 4-Chlor-1-{4-trans-(1,4-dioxa-8-spiro[4,5]decyl)-cyclohexyl)-benzol in 1 ml Tetrahydrofuran sowie 2 ml trockenes Toluol zugegeben und das Reaktionsgemisch auf 100°C geheizt (Oelbadtemperatur). Nach 24 Stunden wird auf -70°C abgekühlt, 0,415 g Trimethylborat zugegeben und langsam auf Raumtemperatur erwärmt. Dann wird 1 ml 1n Salzsäure zugegeben, 30 Minuten gerührt, hierauf zwischen Diäthyläther und Wasser verteilt und die Aetherphase zweimal mit 1n Natronlauge gewaschen. Dabei entsteht in der Aetherphase eine Suspension. Diese wird wiederum mit 1n Salzsäure und dann mehrmals mit Wasser gewaschen, über wasserfreiem Magnesiumsulfat getrocknet und unter vermindertem Druck eingedampft. Der Rückstand ergibt teilweise hydrolysierte 4-[trans-4-(1,4-Dioxa-8-spiro[4,5]decyl)-cyclohexyl]-phenylborsäure. b) 210 mg 4-[trans-4-(1,4-Dioxa-8-spiro[4,5]decyl)-cyclohexyl]-phenylborsäure wird in 2 ml Toluol und 0,8 ml Aethanol unter Erwärmen gelöst. Dann werden 112 mg 1-Brom-3,4-difluorbenzol und 23 mg Tetrakis(triphenylphosphin)palladium (0) sowie 2 ml gesättigte Natriumcarbonatlösung zugegeben. Hierauf wird 15 Stunden bei 80°C (Badtemperatur) geheizt und gut gerührt. Dann wird abgekühlt, zwischen Diäthyläther und Wasser verteilt, die Aetherphase eingedampft und der Rückstand in 5 ml Toluol/Ameisensäure (1:1) aufgenommen. Diese Lösung wird 2 Stunden bei 60°C gerührt, dann abgekühlt, mit Diäthyläther verdünnt, mehrmals mit Wasser und dann mit gesättigter Natriumbicarbonatlösung gewaschen, über wasserfreiem Magnesiumsulfat getrocknet und eingedampft. Chromatographie des Rückstandes an Kieselgel mit Essigester/Petroläther ergibt das trans-4'-(3',4'-Difluor-4-biphenylyl)-[1,1'-bicyclohexyl]-4-on.
c) Eine mit Stickstoff begaste Suspension von 5,707 g trockenem (Methoxymethyl)triphenylphosphoniumchlorid in 35 ml tert.Butylmethyläther wird unter Rühren bei -15°C mit 1,953 g Kalium-tert.butylat versetzt, 30 Minuten gerührt und auf -5°C erwärmt. Zu der orangen Suspension werden innert 10 Minuten 4,1 g trans-4'-(3',4'-Difluor-4-biphenylyl)-[1,1'-bicyclohexyl]-4-on in 20 ml absolutem Tetrahydrofuran und 10 ml tert.Butylmethyläther getropft, das Kühlbad wird entfernt, das Reaktionsgemisch 1,5 Stunden bei Raumtemperatur gerührt, mit 50 ml n-Hexan verdünnt, filtriert und der Filterrückstand mit n-Hexan gewaschen. Das Produkt ergibt nach chromatographischer Reinigung an 120 g Kieselgel bei einem Druck von 0,5 bar mit Methylenchlorid das 4-{4'-(Methoxymethyliden)-[1,1'-bicyclohexyl]-trans-4-yl}-3',4'-difluorbiphenyl.
d) Ein Gemisch von 4,5 g 4-{4'-(Methoxymethyliden)-[1,1'-bicyclohexyl]-trans-4-yl)-3',4'-difluorbiphenyl in 18 ml Tetrahydrofuran und 4,5 ml 2N Salzsäure wird unter Stickstoffbegasung 30 Minuten zum Sieden erhitzt, abgekühlt, mit 150 ml Wasser versetzt und zweimal mit je 100 ml Methylenchlorid extrahiert. Die organischen Phasen werden vereinigt, mit 100 ml 10%iger Natriumchlorid-Lösung gewaschen, über wasserfreiem Natriumsulfat getrocknet und unter vermindertem Druck eingedampft. Dies ergibt ein cis/trans-Gemisch von 4-{4'-(Formyl)-[1,1'-bicyclohexyl]-trans-4-yl}-3',4'-difluorbiphenyl, das in 80 ml Methanol und 8 ml Methylenchlorid gelöst und unter Stickstoff mit 3 Tropfen Triäthylamin und 1,6 ml 20%iger Natronlauge (g/g) 30 Minuten gerührt wird. Die Lösung wird mit 150 ml Wasser versetzt und zweimal mit je 100 ml Methylenchlorid extrahiert. Die organische Phase wird mit 100 ml 10%iger Natriumchloridlösung gewaschen, über wasserfreiem Natriumsulfat getrocknet und ergibt nach dem Eindampfen und nach chromatographischer Reinigung an 120 g Kieselgel bei einem Druck von 0,5 bar mit n-Hexan/Aethylacetat (9:1) und anschliessender Kristallisation aus Methylenchlorid/Hexan bei 0°C reinen 4-{trans-4'-(Formyl)-[bicyclohexyl]-trans-4-yl}-3',4'-difluorbiphenyl.
e) 2,65 g Aethyltriphenylphosphoniumbromid wird unter Argonbegasung in 40 ml tert.Butylmethyläther aufgeschlämmt. Die Suspension wird bei Raumtemperatur mit 797 mg Kalium-tert.butylat versetzt und 1 Stunde gerührt. Anschliessend wird das Gemisch auf 0°C gekühlt, innert 3 Minuten tropfenweise mit einer Lösung von 2,5 g 4-{trans-4'-(Formyl)-[bicyclohexyl]-trans-4-yl}-3',4'-difluorbiphenyl in 15 ml tert.Butylmethyläther versetzt und dann unter Rühren langsam auf Raumtemperatur erwärmen gelassen. Nach 2 Stunden wird die hellgelbe Suspension in Diäthyläther/Wasser verteilt. Die wässrige Phase wird abgetrennt und dreimal mit Diäthyläther extrahiert. Die organischen Phasen werden zweimal mit Wasser gewaschen, über wasserfreiem Magnesiumsulfat getrocknet, filtriert und unter vermindertem Druck eingedampft. Chromatographische Reinigung an Kieselgel mit Aethylacetat/Petroläther ergibt den 4-{trans-4'-(1-Propenyl)-[bicyclohexyl]-trans-4-yl}-3',4'-difluorbiphenyl als Z/E-Gemisch.
f) 2 g 4-{trans-4'-(1-Propenyl)-[bicyclohexyl]-trans-4-yl}-3',4'-difluorbiphenyl (Z/E-Gemisch) werden unter Stickstoffbegasung mit 6 ml Toluol, 0,11 g Natriumbenzolsulfinat und 1 ml 1N Salzsäure versetzt. Das Gemisch wird 15 Stunden bei 50°C gerührt, dann auf 100 ml verdünnte Natriumhydrogencarbonatlösung gegossen und dreimal mit je 50 ml Diäthyläther extrahiert. Die vereinigten organischen Phasen werden mit 100 ml verdünnter Natriumcarbonatlösung und mit 100 ml Wasser gewaschen, über wasserfreiem Magnesiumsulfat getrocknet, filtriert und unter vermindertem Druck eingedampft. Der Rückstand wird an mit Silbernitrat imprägniertem Kieselgel mit Diäthyläther/n-Hexan (1:9) chromatographisch gereinigt. Umkristallisation des erhaltenen Produktes aus Diäthyläther/Methanol ergibt reines 4-{trans-4-(1E-Propenyl)-[1,1'-bicyclohexyl]-trans-4-yl}-3',4'-difluorbiphenyl, Smp. 118,8°C; Klp. (N-I) 332,5°C.

Auf analoge Weise wurden folgende Verbindungen hergestellt:
4-{trans-4-Vinyl-[1,1'-bicyclohexyl]-trans-4-yl}-3',4'-difluorbiphenyl, Smp. (C-N) 98,1°C; Klp. (N-I) >287°C;
4-{trans-4-(1E-Propenyl)-[1,1'-bicyclohexyl]-trans-4-yl}-4'-chlorbiphenyl, Smp. (C-N) 170°C, Klp. (N-I) >450°C;
4-[trans-4-(3-Butenyl)cyclohexyl]-4''-fluor-p-terphenyl, Smp. (C-N) 189,7°C, Klp. (N-I) 327,5°C.

### Beispiel 3

a) 780 mg Natriumborhydrid werden in 30 ml Methanol/Diäthyläther (9:1) gelöst. Diese Lösung wird bei 0°C innert 10 Minuten tropfenweise mit einer Lösung von 10,5 g 3-{trans-4'-(3',4'-Difluor-4-biphenylyl)-[1,1'-bicyclohexyl]-trans-4-yl}-propanol in 40 ml Methanol/Diäthyläther (9:1) versetzt und bei 0°C weiter gerührt. Nach 1,5 Stunden wird das Reaktionsgemisch nochmals mit 200 mg Natriumborhydrid versetzt und weitere 3,5 Stunden bei 0°C gerührt. Anschliessend wird das Reaktionsgemisch mit 20 ml verdünnter Salzsäure angesäuert (pH ca. 2) und dreimal mit Methylenchlorid extrahiert. Die organischen Phasen werden mit verdünnter Salzsäure und zweimal mit Wasser gewaschen, über wasserfreiem Magnesiumsulfat getrocknet und unter vermindertem Druck eingedampft. Das Rohprodukt wird an Kieselgel chromatographisch gereinigt. Hierbei wird 4-{trans-4'-(3-Hydroxypropyl)-[1,1'-bicyclohexyl]-trans-4-yl}-3',4'-difluorbiphenyl erhalten.
b) 1,2 g Natriumhydrid (als 50%ige Suspension in Oel) werden in 20 ml Tetrahydrofuran suspendiert, und die Suspension wird bei Raumtemperatur innert 5 Minuten tropfenweise mit einer Lösung von 6,5 g 4-{trans-4'-(3-Hydroxypropyl)-[1,1'-bicyclohexyl]-trans-4-yl}-3',4'-difluorbiphenyl in 40 ml Tetrahydrofuran versetzt. Das Reaktionsgemisch wird bei Raumtemperatur 5 Minuten weiter gerührt, dann mit 3,75 ml Propyljodid versetzt und noch 2 Stunden unter Rückfluss (Badtemperatur 70°C) gerührt. Anschliessend wird die weisse Suspension in Diäthyläther/Wasser verteilt und dreimal mit Diäthyläther extrahiert. Die organischen Phasen werden zweimal mit Wasser gewaschen, über wasserfreiem Magnesiumsulfat getrocknet, filtriert und unter vermindertem Druck eingedampft. Das Rohprodukt wird an Kieselgel mit Aethylacetat/Petroläther chromatographisch gereinigt und mehrmals aus Methanol/Diäthyläther umkristallisiert. Hierbei wird 4-{trans-4'-(3-Methoxypropyl)-[1,1'-bicyclohexyl]-trans-4-yl}-3',4'-difluorbiphenyl erhalten.

### Beispiel 4

Ein Gemisch aus 3,7 g trans-4'-(1E-Propenyl)-[1,1'-bicyclohexyl]-trans-4-yl malonaldeyd-tetramethylacetal (hergestellt durch Reduktion von trans-4'-(1E-Propenyl)-[1,1'-bicyclohexyl]-trans-4-carbonitril mit Diisobutylaluminiumhydrid, Wittig-Reaktion des erhaltenen Aldehyds mit (Methoxymethyl)triphenylphosphoniumchlorid/Kalium-tert.butylat und bortrifluorid-katalysierter Umsetzung der Methoxyvinyl-Verbindung mit Orthoameisensäuretrimethylester), 0,07 g p-Toluolsulfonsäure-monohydrat und 0,2 ml Wasser wird 2 Stunden bei einer Badtemperatur von 100°C gerührt. Das Reaktionsgemisch wird dann ohne Bad 5 Minuten mit 0,3 g Natriumhydrogencarbonat gerührt, filtriert und der Rückstand mit Methanol nachgewaschen. Das Filtrat wird mit einer Lösung von 1,9 g 4-Chlorbenzamidin-hydrochlorid in 35 ml Methanol und dann mit einer aus 0,42 g Natrium und 10 ml Methanol vorbereiteten Natriummethylat-Lösung versetzt. Das Gemisch wird ca. 16 Stunden bei Raumtemperatur gerührt und die entstandene Suspension durch Zugabe von konzentrierter Salzsäure auf pH 5 gestellt und genutscht. Das so erhaltene rohe 5-{trans-4'-(1E-Propenyl)-[1,1'-bicyclohexyl]-trans-4-yl}-2-(4-chlorphenyl)-pyrimidin wird mit wenig Methanol gewaschen, getrocknet und aus Aethylacetat umkristallisiert.

### Beispiel 5

Zur Untersuchung der Eigenschaften der Verbindungen der Formel I in Gemischen wurden Mischungen von einer "Grundmischung" (GM) folgender Zusammensetzung und jeweils einer Verbindung der Formel I hergestellt:

### Grundmischung (GM)

| Verbindung | Gewichtsprozent |
|---|---|
| 5-(trans-4-Butylcyclohexyl)-2-pyrimidin-carbonitril | 4,12 |
| p-(trans-4-Vinylcyclohexyl)-benzonitril | 8,25 |
| p-[trans-4-(1E-Propenyl)-cyclohexyl]-benzonitril | 8,25 |
| p-[trans-4-(3-Butenyl)-cyclohexyl]-benzonitril | 6,18 |
| p-[trans-4-(3E-Pentenyl)-cyclohexyl]-benzonitril | 7,22 |
| [trans-4'-(3-Butenyl)-[1,1'-bicyclohexyl]-trans-4-yl]-carbonsäure-(p-fluorphenyl)ester | 8,25 |
| 1-[2-(trans-4-Butyl-cyclohexyl)-äthyl]-4-[trans-4-(4-pentenyl)-cyclohexyl]-benzol | 6,18 |
| 4-Aethyl-4'-[trans-4-(3E-Pentenyl)-cyclohexyl]-biphenyl | 4,12 |
| 1-[2-(trans-4-Butyl-cyclohexyl)-äthyl]-4-(trans-4-pentyl-cyclohexyl)-benzol | 8,25 |
| trans-4-Aethoxy-trans-4'-(3-butenyl)-1,1'-bicyclohexyl | 9,28 |
| trans-4-Aethoxy-trans-4'-(3E-pentenyl)-1,1'-bicyclohexyl | 8,25 |
| trans-4-Methoxy-trans-4'-(3E-pentenyl)-1,1'-bicyclohexyl | 8,25 |
| trans-4-Propylcyclohexancarbonsäure-{p-[trans-4-(3-pentenyl)-cyclohexyl]-phenyl}ester | 7,22 |
| 4-[2-(trans-4-Propylcyclohexyl)-äthyl]-4'-(trans-4-pentyl-cyclohexyl)-biphenyl | 4,12 |
| trans-4-Aethoxy-trans-4'-(4-pentenyl)-1,1'-bicyclohexyl | 2,06 |

Die erste Mischung mit einer Verbindung der Formel I ("Mischung 1") bestand aus 97 Gew.-% GM und 3 Gew.-% trans-5-(3-Butenyl)-2-{trans-4'-(4-chlorphenyl)-[1,1'-bicyclohexyl]-trans-4-yl}-1,3-dioxan.

Die zweite ("Mischung 2") bestand aus 97 Gew.-% CM und 3 Gew.-% trans-5-(1E-Propenyl)-2-{trans-4'-(4-chlorphenyl)-[1,1'-bicyclohexyl]-trans-4-yl}-1,3-dioxan.

Die Spannungen in Volt (V) für 10% Transmission (V₁₀) und 50% Transmission (V₅₀) und die Ansprechzeiten in Millisekunden (Einschaltzeit tₒₙ bzw. Ausschaltzeit t_{off}) wurden in einer TN-Zelle (low bias tilt) mit 6 µm Plattenabstand bei 22°C, -20°C und -30°C gemessen; als Betriebsspannung wurde der 2,5-fache Wert der Schwellenspannung gewählt. Ebenfalls gemessen wurden der Schmelzpunkt (Tₘ) und der Klärpunkt (T_{c}) der jeweiligen Mischung.

Die Ergebnisse der Untersuchung sind in der nachfolgenden Tabelle zusammengefasst:

| **Eigenschaften** | **GM** | **Mischung 1** | **Mischung 2** |
|---|---|---|---|
| V₁₀ (V) | 2,153 | 2,150 | 2,145 |
| V₅₀ (V) | 2,526 | 2,534 | 2,535 |
| tₒₙ (ms) bei 22°C | 14,3 | 15,1 | 14,4 |
| tₒₙ (ms) bei -20°C | 177 | 174 | 185 |
| tₒₙ (ms) bei -30°C | 493 | 469 | 529 |
| t_{off} (ms) bei 22°C | 24,1 | 25,3 | 23,8 |
| t_{off} (ms) bei -20°C | 319 | 336 | 345 |
| t_{off} (ms) bei -30°C | 915 | 874 | 973 |
| Tₘ (°C) | <-25 | <-25 | <-25 |
| T_{c} (°C) | 91,2 | 95,0 | 96,8 |

Aus den oben tabellierten Ergebnissen geht hervor, dass die Verbindungen I zwar den Klärpunkt T_{c} bereits in geringen Konzentrationen beträchtlich erhöhen, jedoch nicht zu einer gleichzeitigen Erhöhung der Schwellenspannung V₁₀ und zu keiner (oder zu nur unwesentlicher) Verlängerung der Schaltzeiten führen.

## Patentansprüche

1. Flüssigkristallines Gemisch mit mindestens 2 Komponenten, dadurch gekennzeichnet, daß mindestens eine Komponente eine Verbindung der allgemeinen Formel I ist. 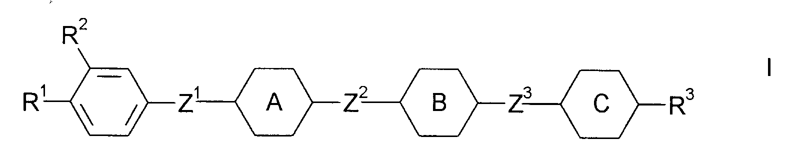 worin
die Ringe A und B unabhängig voneinander trans-1,4-Cyclohexylen, unsubstituiertes oder mit Halogen und/oder Cyano und/oder Methyl mono- oder disubstituiertes 1,4-Phenylen, Pyridin-2,5-diyl oder Pyrimidin-2,5-diyl,
der Ring C trans-1,4-Cyclohexylen oder trans-1,3-Dioxan-2,5-diyl, Z¹ und Z² unabhängig voneinander eine einfache Bindung oder, falls mit mindestens einem gesättigten Ring verknüpft, auch Aethylen,
Z³ eine einfache Bindung oder Aethylen,
R¹ Fluor oder Chlor,
R² Wasserstoff oder Fluor
und
R³ C₂₋₁₂-1E-Alkenyl, C₄₋₁₂-3E-Alkenyl, C₅₋₁₂-Alkenyl oder C₂₋₁₂-Alkoxylalkyl bedeuten, wobei im Falle von Alkoxylalkyl sich nicht mehr als 5 Methylengruppen zwischen dem Sauerstoffatom und dem Ring C befinden,
mit den Maßgaben, daß
(a) falls der Ring B (hetero)aromatisch ist, der Ring A nicht gleichzeitig trans-1,4-Cyclohexylen ist,(b) höchstens zwei mit je einer einfachen Bindung verknüpfte trans-1,4-Cyclohexylengruppen vorhanden sind,
c) höchstens einer der Ringe A und B Pyridin-2,5-diyl oder Pyrimidin-2,5-diyl ist, und
(d) falls gleichzeitig R¹ Fluor und R³ C₂₋₁₂-1E-Alkenyl oder C₄_{*-*}₁₂-3E-Alkenyl bedeuteten, R² ausschließlich für Fluor steht.
wobei der Anteil an Verbindungen der Formel I 2-30 Gew.-% beträgt,
und, daß es eine oder mehrere Verbindungen aus der Gruppe der Verbindungen der allgemeinen Formeln 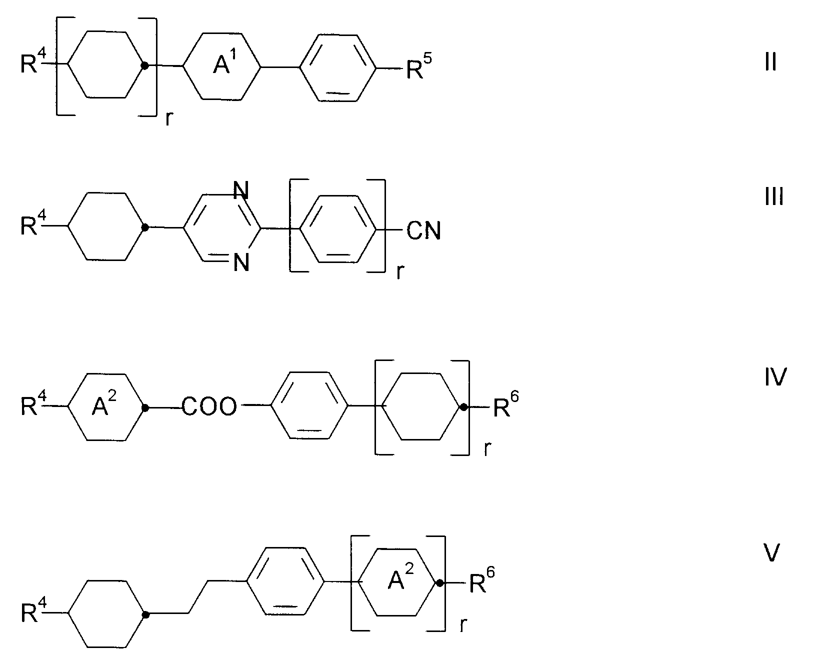 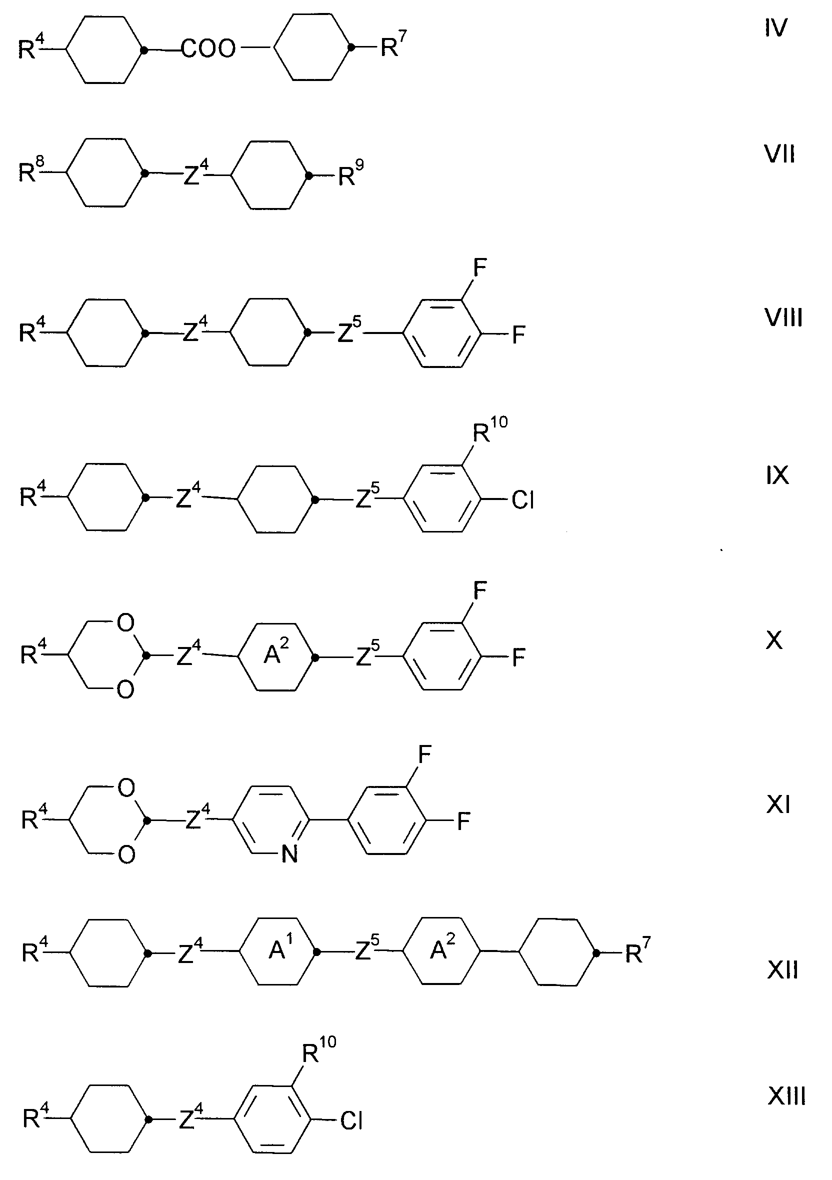 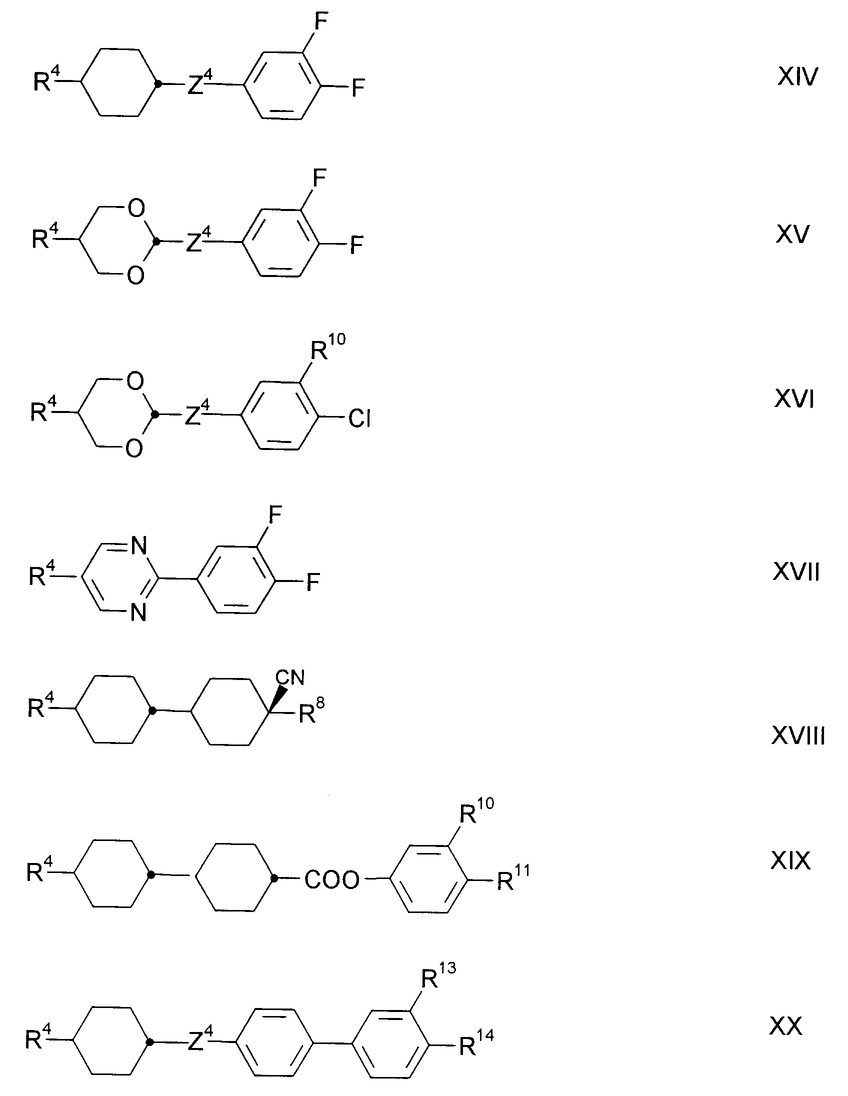
worin
r für die Zahl 0 oder 1 steht;
R⁴ und R⁷ unabhängig voneinander Alkyl, Alkoxylalkyl, 3E-Alkenyl, 4-Alkenyl oder an einem gesättigten Ring auch 1E-Alkenyl bedeutet;
Ring A¹ 1,4-Phenylen, trans-1,4-Cyclohexylen, Pyridin-2,5-diyl, Pyrimidin-2,5-diyl oder trans-1,3-Dioxan-2,5-diyl darstellt;
R⁵ Cyano, Isothiocyanato, Fluor, Alkyl, 3E-Alkenyl, 4-Alkenyl, Alkoxy, 2E-Alkenyloxy, 3-Alkenyloxy oder 1-Alkinyl bezeichnet;
Ring A² 1,4-phenylen oder trans-1,4-Cyclohexylen darstellt;
R⁶ Alkyl, 3E-Alkenyl, 4-Alkenyl oder an einem Cyclohexanring auch 1E-Alkenyl oder an einem Benzolring auch Cyano, Isothiocyanato, Alkoxy, 2E-Alkenyloxy oder 3-Alkenyloxy bedeutet;
R⁸ Alkyl, 1E-Alkenyl, 3E-Alkenyl oder 4-Alkenyl bezeichnet;
Z⁴ und Z⁵ jeweils eine einfache Bindung oder Aethylen darstellt, wobei zwei aromatische Ringe nur durch eine einfache Bindung gebunden sind;
R⁹ Cyano, Alkyl, 1E-Alkenyl, 3E-Alkenyl, 4-Alkenyl, Alkoxy, 2E-Alkenyloxy, 3-Alkenyloxy, Alkoxymethyl oder (2E-Alkenyl)oxymethyl bedeutet;
R¹⁰ Wasserstoff, Fluor oder Chlor bezeichnet;
R¹¹ Fluor, Chlor oder Cyano bedeutet;
R¹² Alkyl, 1E-Alkenyl, 3E-Alkenyl, 3E-Alkenyl oder 4-Alkenyl darstellt;
R¹³ Wasserstoff oder Fluor bedeutet;
und
R¹⁴ Fluor oder Chlor bezeichnet,
enthält.

2. Flüssigkristallines Gemisch nach Anspruch 1, dadurch gekennzeichnet, daß in der Formel I der Ring A trans-1,4-Cyclohexylen oder unsubstituiertes 1,4-Phenylen ist.

3. Flüssigkristallines Gemisch nach Anspruch 1 oder 2, dadurch gekennzeichnet, daß in der Formel I der Ring B trans-1,4-Cyclohexylen ist.

4. Flüssigkristallines Gemisch nach einem der Ansprüche 1 bis 3, dadurch gekennzeichnet, daß in der Formel I Z¹ eine einfache Bindung und Z² und Z³ nicht beide Aethylen sind.

5. Flüssigkristallines Gemisch nach einem der Ansprüche 1 bis 4, dadurch gekennzeichnet, daß in der Formel I R³ Vinyl, 1E-Propenyl, 1E- oder 3-Butenyl, 1E-, 3E- oder 4-Pentenyl, 1E- oder 3E-Hexenyl oder 1E- oder 3E-Heptenyl bedeutet.

6. Elektrooptische Anzeige, dadurch gekennzeichnet, daß sie ein Medium nach einem der Ansprüche 1 bis 5 enthält.

## Claims

1. Liquid-crystalline mixture having at least 2 components, characterized in that at least one component is a compound of the general formula I 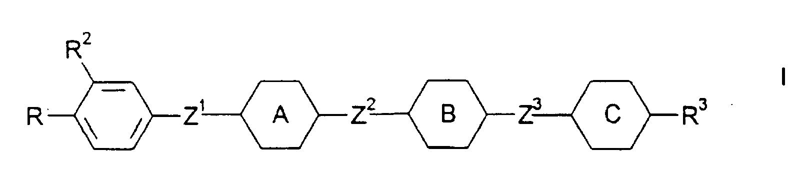 in which
the rings A and B, independently of one another, are trans-1,4-cyclohexylene, unsubstituted or mono- or di-halogen- and/or -cyano- and/or -methyl-substituted 1,4-phenylene, pyridine-2,5-diyl or pyrimidine-2,5-diyl,
the ring C is trans-1,4-cyclohexylene or trans-1,3-dioxane-2,5-diyl,
Z¹ and Z², independently of one another, are a single bond or, if linked to at least one saturated ring, are alternatively ethylene,
Z³ is a single bond or ethylene,
R¹ is fluorine or chlorine,
R² is hydrogen or fluorine,
and
R³ is C₂₋₁₂-1E-alkenyl, C₄₋₁₂-3E-alkenyl, c₅₋₁₂-4-alkenyl or C₂₋₁₂-alkoxyalkyl, where, in the case of alkoxyalkyl, not more than 5 methylene groups are located between the oxygen atom and the ring C,
with the provisos that
(a) if the ring B is (hetero)aromatic, the ring A is not simultaneously trans-1,4-cyclohexylene,
(b) at most two trans-1,4-cyclohexylene groups each linked by a single bond are present,
(c) at most one of the rings A and B is pyridine-2,5-diyl or pyrimidine-2,5-diyl, and
(d) if R¹ is fluorine and simultaneously R³ is C₂₋₁₂-1E-alkenyl or C₄₋₁₂-3E-alkenyl, R² is exclusively fluorine,
where the proportion of compounds of the formula I is 2-30% by weight,
and that the mixture comprises one or more compounds from the group of compounds of the general formulae 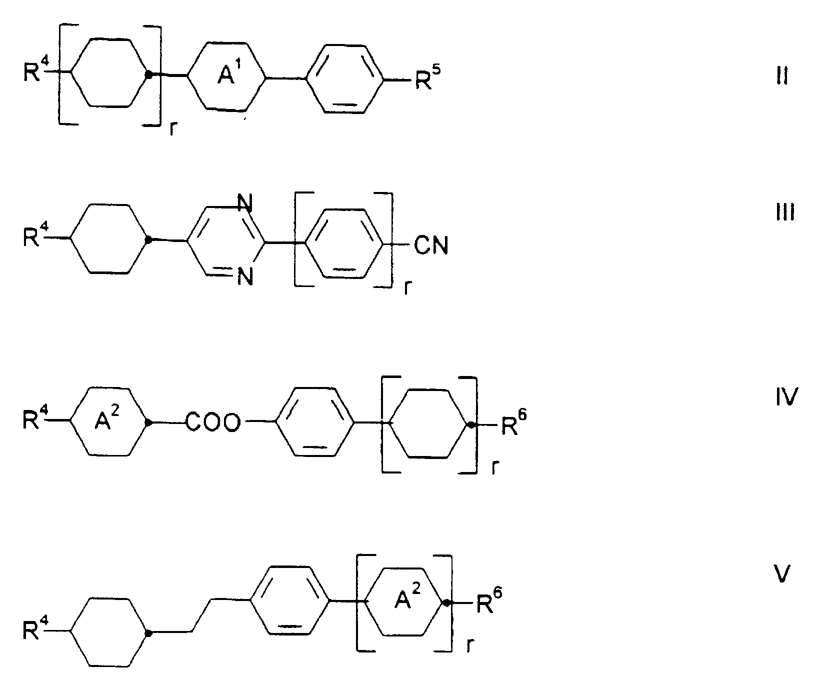 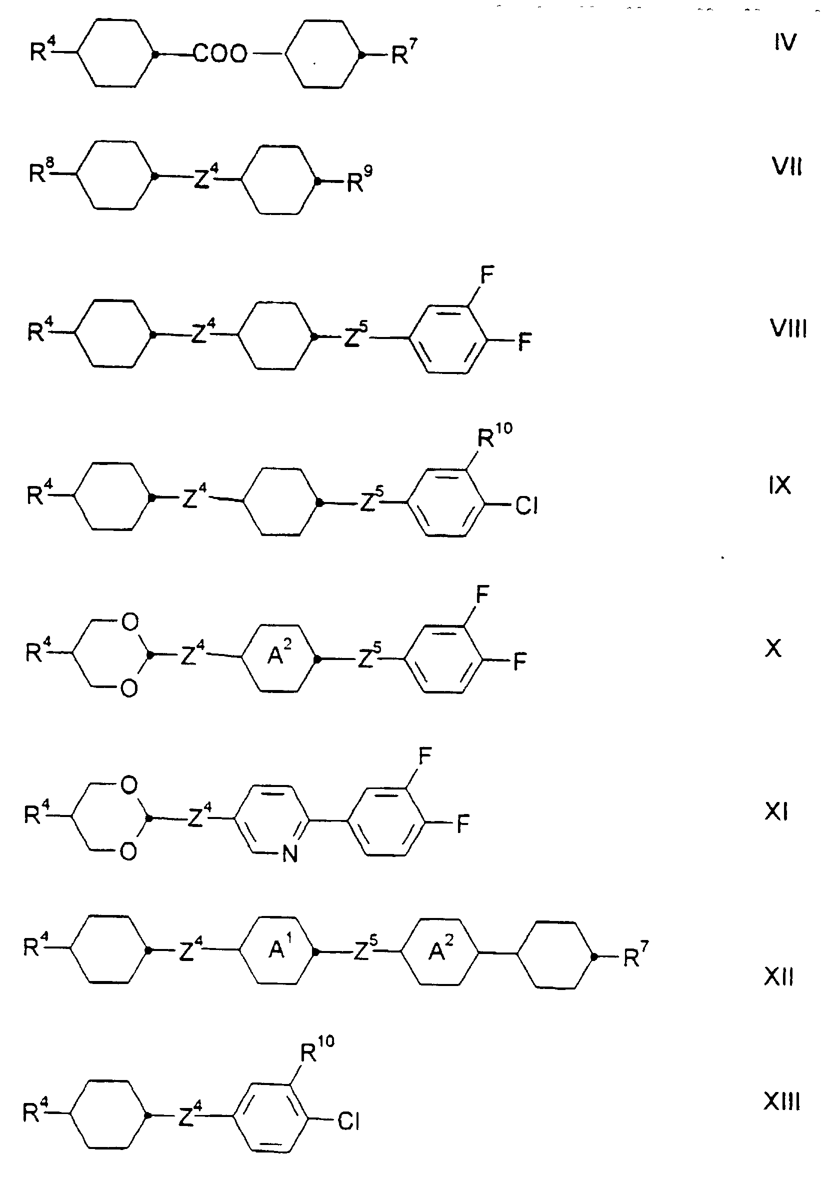 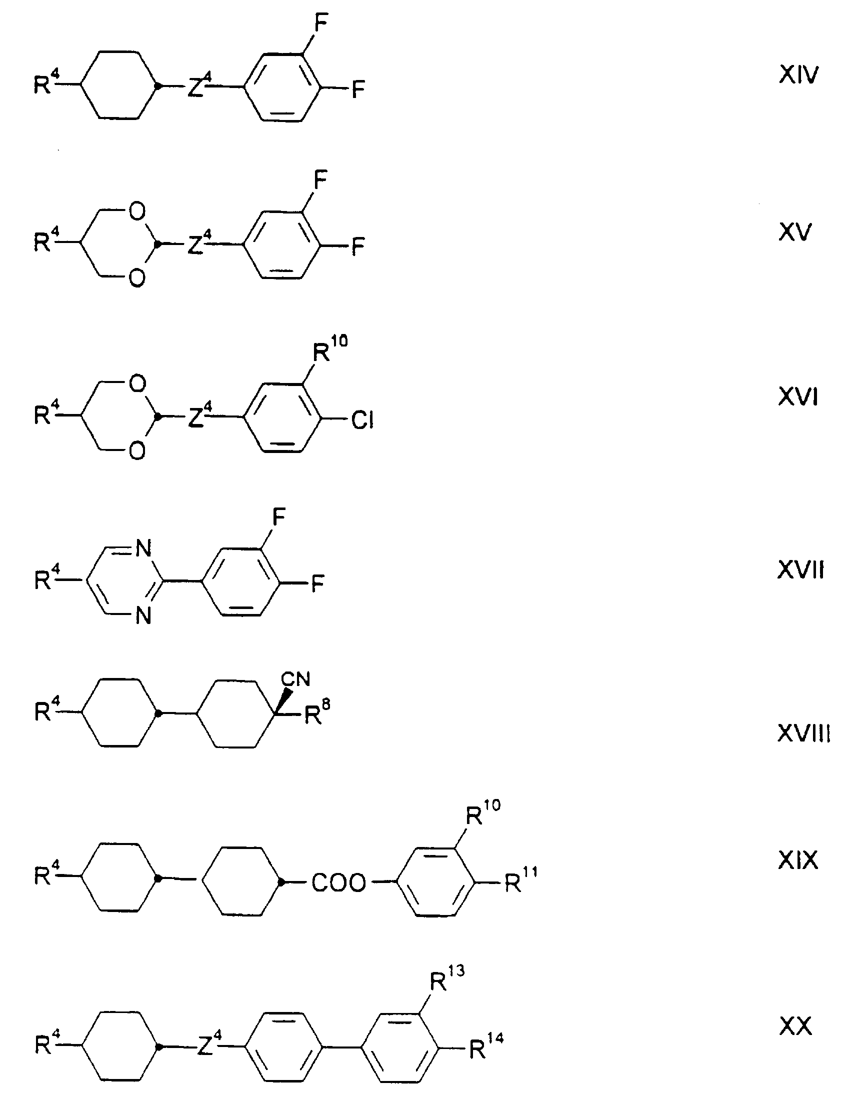 in which
r is the number 0 or 1;
R⁴ and R⁷, independently of one another, are alkyl, alkoxyalkyl, 3E-alkenyl, 4-alkenyl or, on a saturated ring, alternatively 1E-alkenyl;
ring A¹ is 1,4-phenylene, trans-1,4-cyclohexylene, pyridine-2,5-diyl, pyrimidine-2,5-diyl or trans-1,3-dioxane-2,5-diyl;
R⁵ is cyano, isothiocyanato, fluorine, alkyl, 3E-alkenyl, 4-alkenyl, alkoxy, 2E-alkenyloxy, 3-alkenyloxy or 1-alkynyl;
ring A² is 1,4-phenylene or trans-1,4-cyclohexylene;
R⁶ is alkyl, 3E-alkenyl, 4-alkenyl, or, on a cyclohexane ring, alternatively 1E-alkenyl or, on a benzene ring, alternatively cyano, isothiocyanato, alkoxy, 2E-alkenyloxy or 3-alkenyloxy;
R⁸ is alkyl, 1E-alkenyl, 3E-alkenyl or 4-alkenyl;
Z⁴ and Z⁵ are each a single bond or ethylene, where two aromatic rings are only bonded by a single bond;
R⁹ is cyano, alkyl, 1E-alkenyl, 3E-alkenyl, 4-alkenyl, alkoxy, 2E-alkenyloxy, 3-alkenyloxy, alkoxymethyl or (2E-alkenyl)oxymethyl;
R¹⁰ is hydrogen, fluorine or chlorine;
R¹¹ is fluorine, chlorine or cyano;
R¹² is alkyl, 1E-alkenyl, 3E-alkenyl or 4-alkenyl;
R¹³ is hydrogen or fluorine;
and R¹⁴ is fluorine or chlorine.

2. Liquid-crystalline mixture according to Claim 1, characterized in that, in the formula I, ring A is trans-1,4-cyclohexylene or unsubstituted 1,4-phenylene.

3. Liquid-crystalline mixture according to Claim 1 or 2, characterized in that, in the formula I, ring B is trans-1,4-cyclohexylene.

4. Liquid-crystalline mixture according to one of Claims 1 to 3, characterized in that, in the formula I, Z¹ is a single bond, and Z² and Z³ are not both ethylene.

5. Liquid-crystalline mixture according to one of Claims 1 to 4, characterized in that, in the formula I, R³ is vinyl, 1E-propenyl, 1E- or 3-butenyl, 1E-, 3E- or 4-pentenyl, 1E- or 3E-hexenyl, or 1E- or 3E-heptenyl.

6. Electro-optical display, characterized in that it contains a medium according to one of Claims 1 to 5.

## Revendications

1. Mélange cristallin liquide comportant au moins deux composants, caractérisé en ce qu'au moins un composant est un composé de formule générale I : 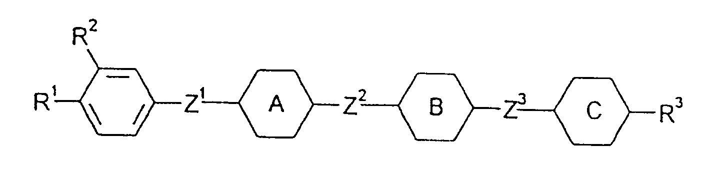 où
◆ les cycles A et B représentent, indépendamment l'un de l'autre, le trans-1,4-cyclohexylène, les 1,4-phénylène, pyridine-2,5-diyle ou pyrimidine-2,5-diyle non substitués ou mono- ou disubstitués par un halogène et/ou le cyano et/ou le méthyle,
◆ le cycle C représente le trans-1,4-cyclohexylène ou le trans-1,3-dioxane-2,5-diyle,
◆ Z¹ et Z² représentent, indépendamment l'un de l'autre, une liaison simple ou, s'ils sont reliés à au moins un cycle saturé, également l'éthylène,
◆ Z³ représente une liaison simple ou l'éthylène,
◆ R¹ le fluor ou le chlore,
◆ R² l'hydrogène ou le fluor et
◆ R³ un 1E-alcényle en C₂-C₁₂, un 3E-alcényle en C₄-C₁₂, un 4-alcényle en C₅-C₁₂ ou un alcoxyalkyle en C₂-C₁₂, dans le cas d'un alcoxyalkyle pas plus de 5 groupes méthylène se trouvant entre l'atome d'oxygène et le cycle C,
sous réserve :
(a) que lorsque le cycle B est (hétéro)aromatique, le cycle A n'est pas en même temps le trans-1,4-cyclohexylène,
(b) qu'au plus deux groupes trans-1,4-cyclohexylène reliés par une liaison simple soient présents,
(c) qu'au plus l'un des cycles A et la est le pyridine-2,5-diyle ou le pyrimidine-2,5-diyle et
(d) que dans le cas où en même temps R¹ représente le fluor et R³ un 1E-alcényle en C₂-C₁₂ ou un 3E-alcényle en C₄-C₁₂, R² représente exclusivement le fluor,
la proportion de composés de formule I étant comprise entre 2 et 30 % en poids et en ce qu'il contient un ou plusieurs composés du groupe des composés de formules générales : 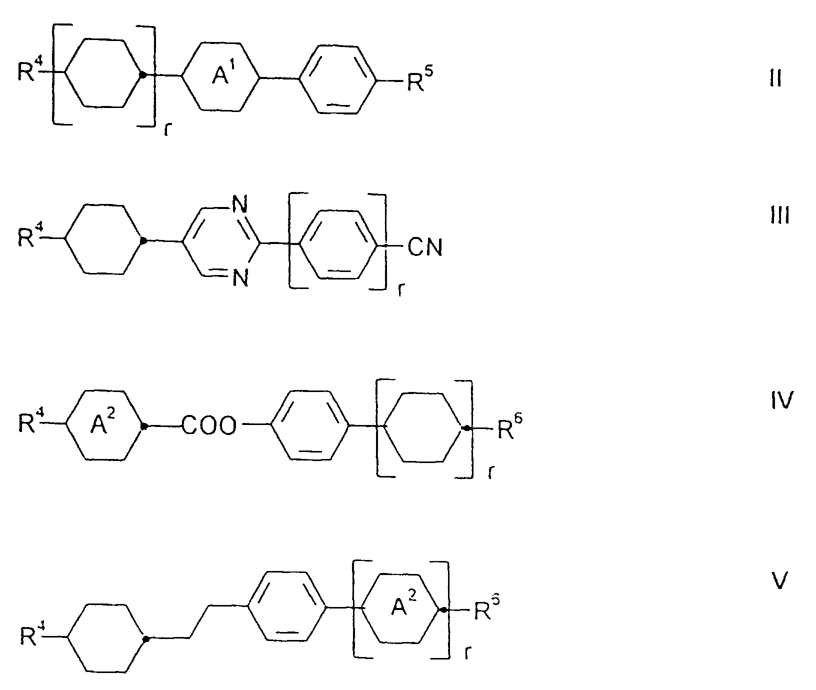 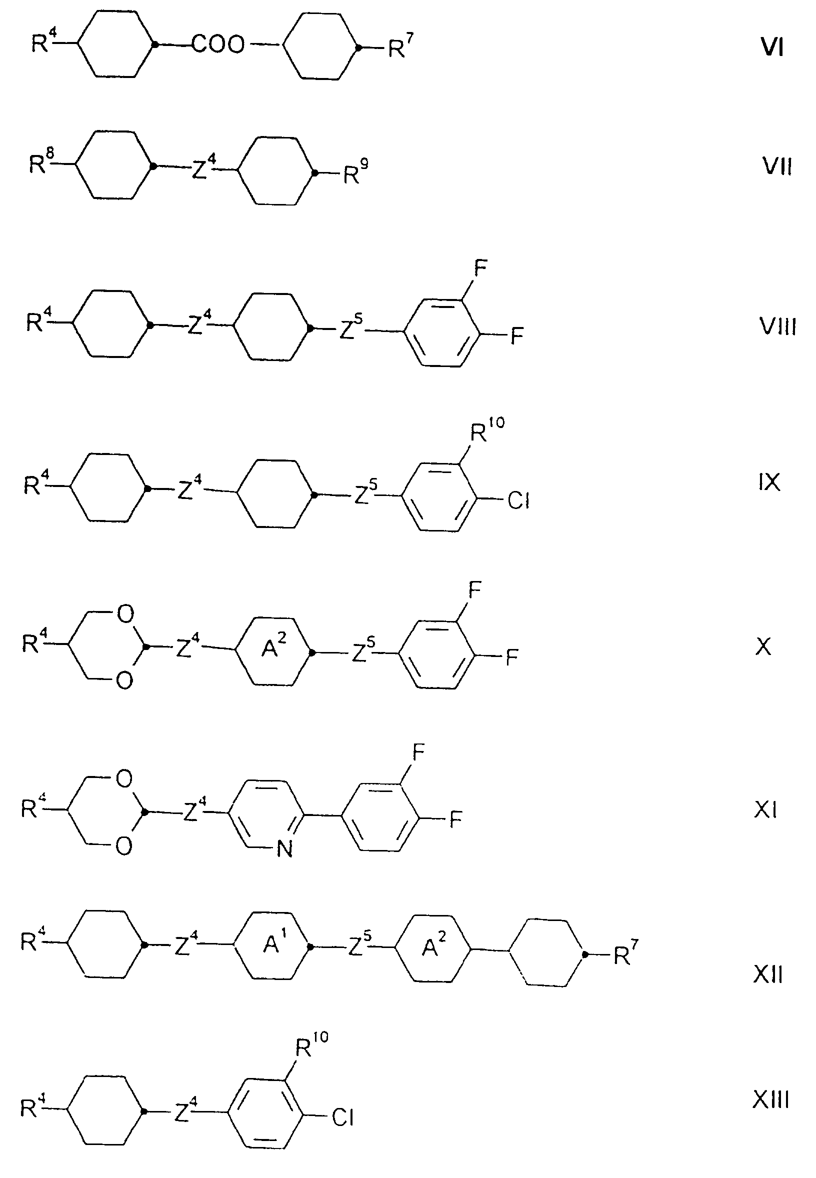 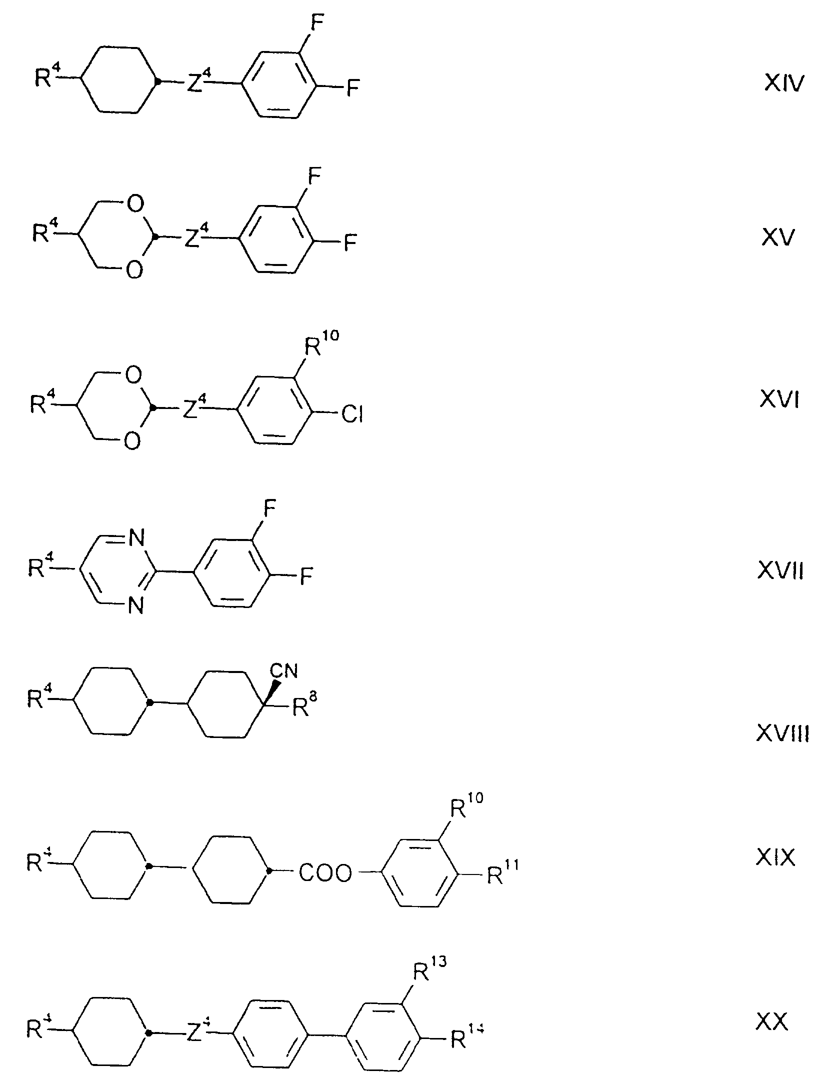 où
◆ r est égal à 0 ou 1,
◆ R⁴ est R⁷ représentent, indépendamment l'un de l'autre, un alkyle, un alcoxyalkyle, un 3E-alcényle, un 4-alcényle ou sur un cycle saturé également un 1E-alcényle,
◆ le cycle A¹ représente le 1,4-phénylène, le trans-1,4-cyclohexylène, le pyridine-2,5-diyle, le pyrimidine-2,5-diyle ou le trans-1,3-dioxane-2,5-diyle,
◆ R⁵ le cyano, l'isothiocyanato₁ le fluor, un alkyle, un 3E-alcényle, un 4-alcényle, un alcoxy, un 2E-alcényloxy, un 3-alcényloxy ou un 1-alcynyle,
◆ le cycle A² le 1,4-phénylène ou le trans-1,4-cyclohexylène,
◆ R⁶ un alkyle, un 3E-alcényle, un 4-alcényle ou sur un cycle cyclohexane également un 1E-alcényle ou sur un cycle benzénique également le cyano, l'isothiocyanato, un alcoxy, un 2E-alcényloxy ou un 3-alcényloxy,
◆ R⁸ un alkyle, un 1E-alcényle, un 3E-alcényle ou un 4-alcényle,
◆ Z⁴ et Z⁵ représentent une liaison simple ou l'éthylène, deux cycles aromatiques n'étant liés que par une liaison simple,
◆ R⁹ représente le cyano, un alkyle, un 1E-alcényle, un 3E-alcényle, un 4-alcényle, un alcoxy, un 2E-alcényloxy, un 3-alcényloxy, un alcoxyméthyle ou un (2E-alcényl)oxyméthyle,
◆ R¹⁰ l'hydrogène, le fluor ou le chlore,
◆ R¹¹ le fluor, le chlore ou le cyano,
◆ R¹² un alkyle, un 1E-alcényle, un 3E-alcényle, un 3E-alcényle ou un 4-alcényle,
◆ R¹³ l'hydrogène ou le fluor, et
◆ R¹⁴ le fluor ou le chlore.

2. Mélange cristallin liquide selon la revendication 1, caractérisé en ce que dans la formule I le cycle A est le trans-1,4-cyclohexylène ou le 1,4-phénylène non substitué.

3. Mélange cristallin liquide selon la revendication 1 ou 2, caractérisé en ce que dans la formule I le cycle B est le trans-1,4-cyclohexylène.

4. Mélange cristallin liquide selon l'une des revendications 1 à 3, caractérisé en ce que dans la formule I Z¹ est une liaison simple et Z² et Z³ ne sont pas tous les deux l'éthylène.

5. Mélange cristallin liquide selon l'une des revendications 1 à 4, caractérisé en ce que dans la formule I R³ représente le vinyle, le 1E-propényle, le 1E-ou le 3-butényle, le 1E-, le 3E- ou le 4-pentényle, le 1E- ou le 3E-hexényle ou le 1E- ou le 3E-heptényle.

6. Afficheur électro-optique caractérisé en ce qu'il contient un milieu selon l'une des revendications 1 à 5.
